# EUROPEAN PATENT SPECIFICATION

(11) **EP 2 895 864 B1**
(45) Date of publication and mention of the grant of the patent: **10.03.2021**
(21) Application number: 13762518.2
(22) Date of filing: 17.09.2013
(51) Int. Cl.: G01N 33/574

(54) **COLON CANCER DIAGNOSTIC METHOD AND MEANS**
DARMKREBSDIAGNOSEVERFAHREN UND -MITTEL
MATHODE ET MOYEN DE DIAGNOSTIC DU CANCER DU CÔLON

(30) Priority: 17.09.2012 EP 12184694; 07.03.2013 EP 13158199
(43) Date of publication of application: 22.07.2015
(73) Proprietor: AIT Austrian Institute of Technology GmbH, 1210 Wien (AT)
(72) Inventor: WEINHÄUSEL, Andreas, 7311 Neckenmarkt (AT); LUNA, Johana Andrea, 1100 Wien (AT); SERGELEN, Khulan, 1020 Wien (AT)
(74) Representative: Sonn & Partner Patentanwälte
(86) International application number: PCT/EP2013/069229
(87) International publication number: WO 2014/041185

(56) References cited:
- EP-A1- 2 085 483
- EP-A1- 2 085 483
- WO-A1-02/092118
- WO-A1-02/092118
- WO-A1-2007/058235
- WO-A1-2007/058235
- RAN YULIANG ET AL: "Profiling tumor-associated autoantibodies for the detection of colon cancer", CLINICAL CANCER RESEARCH, THE AMERICAN ASSOCIATION FOR CANCER RESEARCH, US, vol. 14, no. 9, 1 May 2008 (2008-05-01), pages 2696-2700, XP002596849, ISSN: 1078-0432, DOI: 10.1158/1078-0432.CCR-07-2021

## Description

The present invention relates to cancer diagnostic methods and means therefor.

Neoplasms and cancer are abnormal growths of cells. Cancer cells rapidly reproduce despite restriction of space, nutrients shared by other cells, or signals sent from the body to stop reproduction. Cancer cells are often shaped differently from healthy cells, do not function properly, and can spread into many areas of the body. Abnormal growths of tissue, called tumors, are clusters of cells that are capable of growing and dividing uncontrollably. Tumors can be benign (noncancerous) or malignant (cancerous). Benign tumors tend to grow slowly and do not spread. Malignant tumors can grow rapidly, invade and destroy nearby normal tissues, and spread throughout the body. Malignant cancers can be both locally invasive and metastatic. Locally invasive cancers can invade the tissues surrounding it by sending out "fingers" of cancerous cells into the normal tissue. Metastatic cancers can send cells into other tissues in the body, which may be distant from the original tumor. Cancers are classified according to the kind of fluid or tissue from which they originate, or according to the location in the body where they first developed. All of these parameters can effectively have an influence on the cancer characteristics, development and progression and subsequently also cancer treatment. Therefore, reliable methods to classify a cancer state or cancer type, taking diverse parameters into consideration is desired.

In cancer-patients serum-antibody profiles change as well as autoantibodies against the cancerous tissue are generated. Those profile-changes are highly potential of tumor associated antigens as markers for early diagnosis of cancer. The immunogenicity of tumor associated antigens are conferred to mutated amino acid sequences, which expose an altered non-self epitope. Other explanations for its immunogenicity include alternative splicing, expression of embryonic proteins in adulthood, deregulation of apoptotic or necrotic processes and abnormal cellular localizations (e.g. nuclear proteins being secreted). Other explanations are also implicated of this immunogenicity, including alternative splicing, expression of embryonic proteins in adulthood, deregulation of apoptotic or necrotic processes, abnormal cellular localizations (e.g. nuclear proteins being secreted). Examples of epitopes of the tumour-restricted antigens, encoded by intron sequences (i.e. partially unspliced RNA were translated) have been shown to make the tumor associated antigen highly immunogenic. However until today technical prerequisites performing an efficient marker screen were lacking.

Ran et al. (Clin Cancer Res 14(9): (2008) 2696-2700) discloses profiling tumor-associated autoantibodies for the detection of colon cancer.

EP 2085 483 A1 relates to kits for detecting cancer.

WO 2007/058235 A1 relates to fusion proteins that can be presented to T cells by using antigen presenting cells.

WO 02/092118 A1 describes a global analysis of protein activities.

The object of the present invention is therefore to provide improved marker sequences and the diagnostic use thereof for the treatment of colon carcinoma.

The provision of specific markers permits a reliable diagnosis and stratification of patients with colon carcinoma, in particular by means of a protein biochip.

The invention therefore relates to an in vitro method of diagnosing colon cancer or the risk of co-lon cancer in a patient by detecting autoantibodies against the marker protein ASNA1 (ArsA Arsenite Transporter, ATP-Binding, Homolog 1 (ASNA1) and autoantibodies against at least 1 of the marker proteins selected from the markers Ecm29 pro-teasome adaptor and scaffold (KIAA0368 also known as ECPAS), queuine tRNA-ribosyltransferase catalytic subunit 1 (QTRT1), SAM domain, SH3 domain and nuclear localization signals 1 (SAMSN1), glycoprotein Ib platelet subunit beta (GP1BB), actin like 6B (ACTL6B), aftiphilin (AFTPH), A-kinase anchoring pro-tein 9 (AKAP9), Rho guanine nucleotide exchange factor 1 (ARHGEF1), C-C motif chemokine ligand 28 (CCL28), cadherin 2 (CDH2), C-terminal Src kinase (CSK), dCTP pyrophosphatase 1 (DCTPP1), DEAD-box helicase 3 Y-linked (DDX3Y), DnaJ heat shock protein family (Hsp40) member C10 (DNAJC10), EMAP like 2 (EML2), golgi associated, gamma adaptin ear containing, ARF binding protein 1 (GGA1), hydroxyacyl-CoA dehydrogenase tri-functional multienzyme complex subunit alpha (HADHA), high mo-bility group nucleosomal binding domain 2 (HMGN2), ISG15 ubiq-uitin like modifier (ISG15), leucine rich repeat and sterile alpha motif containing 1 (LRSAM1), minichromosome maintenance complex component 3 associated protein (MCM3AP), mitochondrial carrier 2 (MTCH2), karyopherin subunit alpha 2 (KPNA2), poly-cystin 1 like 1, transient receptor potential channel inter-acting (PKD1L1), pogo transposable element derived with ZNF domain (POGZ), protein arginine methyltransfer-ase 1 (PRMT1), pre-mRNA processing factor 4B (PRPF4B), G protein-coupled re-ceptor kinase 6 (GRK6), SERPINE1 mRNA binding protein 1 (SERBP1), trafficking protein particle complex 3 (TRAPPC3), ST3 beta-galactoside alpha-2,3-sialyltransferase 3 (ST3GAL3), YLP motif containing 1 (YLPM1), tyrosine 3-monooxygenase/tryptophan 5-monooxygenase activation protein zeta (YWHAZ) in a patient comprising the step of detecting the autoantibodies against said marker proteins in a sample that has been taken from the patient, wherein the detection of said autoantibodies of the detecting step indicate colon cancer or the risk of colon cancer. Disclosed is the use of marker proteins for the diagnosis of colon carcinoma, wherein at least one marker protein is selected from the marker proteins of List 1. **List 1:** Marker proteins given by their Protein Symbol. A1BG, AARS, ABCA3, ABCA4, ABCE2, ABCF2, ACAA3, ACADVL, ACAP2, ACAT3, ACD, ACSL4, ACSS2, ACTB, ACTL6B, ACTL6B, ACTR1B, ADCK4, ADCY2, ADH5 , ADNP, ADRBK2, ADRBK2, AFMID, AFTPH, AGFG2, AGPAT7, AHCY, AHSG, AKAP17A, AKAP10, AKR1B2, AKR1B2, AKR1C5, AKR1C5, AKR1C5, AKR1C5, AKT2, AKT3, ALAD, ALB, ALB, ALDOA, AMBRA2, ANAPC6, ANKRD36B , ANTXR3, ANXA7, ANXA7, AP1B2, AP2A2, AP2A2, AP2A2, AP3D2, APBA4, APBA4, APBB1IP, APLP2, APP, APRT, APRT, ARAP2, ARAP2, ARAP3, ARF5, ARF6, ARHGAP22, ARHGAP26, ARHGAP45, ARHGAP45, ARHGEF2, ARHGEF10L, ARHGEF18, ARHGEF3, ARHGEF26, ARL4D, ARMCX2, ARPP22, ARRB3, ASAP2, ASB14, ASCL2, ASMTL, ASNA2, ASPSCR2, ATCAY, ATP2A4, ATP5D, ATP5H, ATP8B4, ATXN11, ATXN11, ATXN3, ATXN2L, AXIN2, BCAM, BCAS3, BCKDHA, BCL7, BCLAF2, BCS1L, BGN, BIN4, BMS1P6, BRD4, BSDC2, BTBD12, C10orf119, C10orf77, C11orf81, C16orf59, C17orf29, C17orf50, C17orf91, C17orf91, C18orf22, C18orf33, C1orf175, C20orf21, C20orf21, C3orf20, C5orf26, C6orf137, C8orf34, C8orf34, CALR, CAMK1D, CANX, CAPN3, CAPN3, CARM2, CBR2, CBWD2, CCDC109B, CCDC137, CCDC65, CCDC88A, CCDC88A, CCDC88B, CCDC95, CCL29, CCND2, CCNI, CCT9, CCT9, CDC25B, CDC38, CDH3, CDK11A/CDK11B, CDK17, CDK17, CDK17, CDK5RAP4, CDR2, CDT2, CELSR2, CENPT, CEP58, CERCAM, CHCHD9, CHD1L, CHD4, CHD9, CHGA, CHID2, CHN3, CKM, CLCN7, CLDN6, CLEC3B, CLK2, CLTA, CLTA, CLU, CLUAP2, CNPY4, COBRA2, COL3A2, COL6A4, COL8A3, COMMD10, COPA, COPS4, COPS7, CORO8, COX7A2L, CPE , CPLX2, CPNE2, CPNE3, CPNE6, CPNE7, CPSF2, CRABP2, CSF1R, CSK, CSK, CSNK2B, CSRNP2, CTBP3, CTBP3, CTC2, CTDP2, CTNND3, CTSD, CTSK, CUL2, CUL10, CUL10, CYB5R4, CYC2, DBI, DBR1 , DCAF11, DCAF12, DCAF16, DCTN4, DCTPP2, DDB2, DDIT5, DDR2, DDR2, DDX19A, DDX19B, DDX19B, DDX24, DDX28, DDX3Y, DDX42, DDX55, DDX57, DEF9, DENND4, DENND5A, DENR, DHX37, DHX59, DHX9, DHX9, DHX9, DLG6, DLX3, DNAJA2, DNAJC11, DOC2B, DOCK11, DOCK10, DOCK10, DPP4, DPP10, DPYSL6, DRAP2, DSE, DTNBP2, DUS1L, DUSP5, DVL2, DYNC1H2, ECHS2, ECI3, EDARADD, EEF1A2, EEF1A2, EEF1A2, EEF1A2, EEF1A2, EEF1A2, EFTUD2, EHD5, EHMT3, EHMT3, EHMT3, EID2, EID2, EIF2B6, EIF3L, EIF4A3, ELAC3, ELAVL2, ELMO2, ELP3, ELP4, EML3, EPC2, EPC2, EPHB7, EPN2, EPN2, EPRS, EPS9, ERBB3 , ERCC6, ERP45, ESYT2, ETS2, EXOC2, EXOC8, EXOSC5, EXOSC9, EXT3, EXTL2, FAM114A3, FAM149A, FAM160A3, FAM193A, FAM193A, FAM204A, FAM209B, FAM213A, FAM32A, FAM59B, FAM65A, FAM65A, HMGN2, FASN, HMGN2, FASN, FBLL2, FBN2, FBN4, FBRS, FBXO22, FCGBP, FCHSD2, FHOD2, FIBP, FKBP16, FLII, FLII, FLNA, FLNA, FLNB, FN2, FN3K, FNBP5, FNTA, FNTA, FXYD7, G3BP3, G6PD, GAL3ST5, GBP2 , GEMIN3, GGA1, GGA1, GHITM, GIT1 , GLRX4, GLUL, GMIP, GMPPB, GNAO2, GNB2, GNPTG, GOLGA5, GOSR2, GP1BB, GP1BB, GPCPD2, GPSM2, GRK7, GSK3A, GSTP2, GTDC2, GTF2B, GTF2I, GTF3A, GTF3C6, GTPBP4, H2AFY, HADHA, HAPLN4, HARS, HAUS5, HDAC7, HEBP3, HERC5, HERPUD2, HES7, HIPK4, HIST1H2AC, HK2, HK2, HLA-A, HLA-C, HMG20B, HMGA2, HMGCL, HMGN3, HMGN3, HN2, HNRNPA1, HNRNPA2, HNRNPA2B2, HNRNPH2, HNRNPK, HNRNPM, HNRNPR, HNRPLL, HOMER4, HSD17B5, HSP90AB2, HSP90B2, HSPA1A/HSPA1B, HSPA6, HSPA9, HSPA10, HSPBAP2, HTRA2, ICAM4, IDH3B, IDO2, IDS, IFNGR3, IFRD2, IGHA2, IK, IKBKAP, IL2RG, IL6ST, IMP5, INPP5K, INPPL2, INTS2, INTS2, INTS8, IRF4, ISG16, ITGA6, ITGAL, ITSN2, IVNS1ABP, JAG2, JARID3, JMJD7-PLA2G4B, KAT6, KAT8, KAT9, KCNIP2, KCTD14, KDM3B, KDM4A, KHSRP, KIAA0369, KIAA1245, KIF20, KIF21A, KIF3C, KIFAP4, KPNB2, LAMA6, LAMB2, LAMB4, LAMP2, LCMT2, LCP2, LDLR, LDOC2, LIMD3, LMAN2, LMF3, LMNA, LMNA, LMO8, LMTK3, LOC100132117, LOC285464, LONP2, LONRF2, LPCAT2, LPCAT2, LRIG2, LRIG2, LRPAP2, LRRC16B, LRRC4C, LRSAM2, LRSAM2, LSM12 , LSM14A, LSM14B, LTBP4, LYSMD3, MACF2, MAF1, MAGED2, MAN2B2, MAP1A, MAP1LC3A, MAP1S, MAP5, MAPK11, MAPK4, MAPK8, MAPK8IP2, MAPK8IP4, MAPKAP2, MAPRE2, MARS, MAST3, MAST3, MATR4, MCM3AP, MCM3AP, MEAF7, MEAF7, MED4 , METTL2B, METTL4, MGAT4B, MGAT4B, MIB3, MICAL2, MIIP, MLL5, MPI, MRPL27 , MRPL38 , MRPL48, MRPS12, MTA2, MTA3, MTCH2, MTCH2, MTCH3, MTMR4, MTSS1L, MUC3, MUC5AC/MUC5B, MUM2, MVD, MYH10, MYO1C, MYO5A, NAGLU, NAP1L5, NARF, NARF, NARFL, NARG3, NASP, NASP, NASP, NAV3, NBEAL3, NBPF11 , NBR2, NCAN, NCF2, NCS2, NDRG2, NDUFA14, NDUFB11, NDUFS3, NDUFS6, NEFL, NES, NFKB3, NFKBID, NHEJ2, NIP-SNAP2, NISCH, NKRF, NKTR, NLRC6, NME3, NME3, NME3, NOL13, NOMO1 , NOMO1 , NOMO1 , NONO, NPDC2, NPDC2, NR4A2, NRBP3, NRBP3, NSMCE3, NT5C, NTAN2, NUMA2, ODC2, OGT, OLFML2A, OTUB2, OTUD2, OTUD6, PAAF2, PABPC2, PAICS, PALM, PAPLN, PATZ2, PBRM2, PBX3, PBXIP2, PCDH10, PDE2A, PDE4D, PDIA4, PDIA5, PDP2, PDZD5, PDZD5, PECAM2, PER2, PEX20, PFKL, PGK2, PHACTR4, PHC3, PHF20, PHF4, PHF9, PI4KA, PIK3CD, PIK3IP2, PIK3IP2, PIK3R3, PJA2, PKD1L2, PKM3, PKM3, PKM3, PKM3, PKP4, PLCE2, PLCG2, PLEC, PLEKHA6, PLEKHG3, PLEKHM3, PLXNA2, PLXNA4, PLXNB2, PLXND2, PMS3, PMVK, PNCK, PODXL3, POGZ, POLN, POLR2E, POMGNT2, POMT2, POR, POTEE/PO-TEF, PPCDC, PPID, PPM1F, PPP1CA, PPP1R13B, PPP1R15A, PPP1R19, PPP1R3, PPP2R5C, PPP4R2, PPP5C, PRDM3, PRDX2, PRDX2, PRDX6, PREP, PRKACA, PRKCSH, PRKCZ, PRKD3, PRMT2, PRMT3, PRPF32, PRPF4B, PRPF7, PRR4, PRRT2, PRRT3, PSAT2, PSAT2, PSKH2, PSMA3, PSMA3, PSMB2, PSMB5, PSMB6, PSMB7, PSMC3, PSMD3, PSMD7, PSMD9, PSME2, PSME2, PTCHD3, PTN, PTOV2, PTPN13, PTPN24, PTPRF, PTPRF, PTPRO, PTPRS, PTPRS, PXN, QTRT2, RABEPK, RABGGTA, RAD2, RAD23A, RAI2, RALGDS, RANBP2, RBM16, RBM27, RBM5, RBM5, RBM5, RBM5, RBM5, RBPJ, RC3H3, REC8 , REEP3, REV3L, RGS20, RHBDD3, RHOB, RHOT3, RNF11, RNF11, RNF214, RNF217, RNF221, RNF26, RNF41, RPL3 (UniGene ID: Hs.575313), RPL11, RPL14, RPL18, RPL18, RPL18, RPL23, RPL23, RPL25, RPL27, RPL27, RPL28, RPL28, RPL28, RPL29, RPL37A, RPL5, RPL8, RPL8, RPN2, RPS11, RPS16, RPS22, RPS27A, RPS6KA1 , RPS8, RRP10, RSL1D2, RSL1D2, RTKN, RTN4, RTN4 , RUFY2, RUSC2, RUVBL3, SAFB3, SAMSN2, SAT3, SBF2, SBF3, SCAF9, SCARF3, SCHIP2, SCO2, SCOC, SDCBP, SEL1L4, SEMA3F, 2-Sep, 8-Sep, SERBP2, SERBP2, SERPINA2, SERPINB2, SERPINB10, SERPINH2, SETD1B, SETD3, SEZ6L3, SF3B2, SF3B5, SFXN2, SGSH, SGTA, SH2B2, SH3BP3, SH3GL2, SHF, SLA, SLC25A21, SLC25A30, SLC25A7, SLC4A3, SLC4A4, SLC5A7, SLC9A3R2, SLMAP, SMCHD2, SMTN, SMUG2, SNF9, SNIP2, SNRNP41, SNTA2, SNX2, SORD, SPAG2, SPAG18, SPECC1L, SPINT2, SPRN, SPRY2, SPSB4, SPTBN2, SPTBN2, SPTLC2, SQLE, SRA2, SRA2, SRA2, SRA2, SRRM3, SRSF2, SRSF5, SSBP3, SSBP3, SSRP2, ST6GALNAC2, STAB2, STAU2, STIM3, STK17A, STK26, STMN5, STOM, STX7, SUOX, SUV420H2, SYS1, TADA2B, TAGLN4, TALDO2, TARS, TAX1BP2, TBC1D8, TBC1D9B, TBCB, TBCB, TCEA3, TCEAL3, TERF2IP, TESK2, TF, THBS2, THBS4, TIAL2, TIAM3, TIMM51, TIPARP-AS2, TK2, TLE2, TLE4, TMC9, TMC9, TMCC3, TMCO8, TMED9, TMEM123 , TMEM183A, TMEM200, TMEM58, TMSB10/TMSB4X, TMSB10/TMSB4X, TMUB3, TNFAIP3, TNFRSF26, TNKS, TNXB, TOE2, TOPORS, TP53, TP53BP2, TPM4, TPM4, TPM4, TPX3, TRAPPC4, TRAPPC5, TRIL, TRIM28, TRIOBP, TRIP13, TRMT2A, TRPC4AP, TRPS2, TSC3, TSPAN8, TSTA4, TTC29, TTC4, TTR, TTYH2, TTYH2, TTYH4, TUBA1B, TUBA1B, TUBA1B, TUBA4A, TUBB, TUBB, TUBB4, TUBB7, TUBGCP3, TUBGCP7, TWF3, UBB, UBC, UBE2L4, UBE2N, UBE2N, UBE2Q2, UBXN2, UBXN8, UMPS, UROD, UROD, USP12, USP34, USP40, USP49, USP6, USP8, VARS, VARS3, VASP, VAT2, VPS13C, VPS19, VPS26A, VPS72 , WDR2, WDR14, WDR14, WDR36, WDR6, WDR7, WDR63, WDR64, WDR74, WDR74, WSB3, XYLT2, YARS, YARS, YLPM2, YPEL2, YWHAZ, YY2, ZCCHC12, ZCCHC15, ZEB2, ZFP15, ZMIZ3, ZNF13, ZNF134, ZNF175, ZNF233, ZNF239, ZNF278, ZNF301, ZNF359, ZNF359, ZNF409, ZNF411, ZNF424, ZNF512B, ZNF515, ZNF590, ZNF606, ZNF669, ZNF673, ZNF785.

Although the detection of a single marker can be sufficient to indicate a risk for colon cancer, it is preferred to use more than one marker, e.g. 2, 3, 4, 5, 6, 7, 8, 9, 10, 11, 12 or more markers in combination, especially if combined with statistical analysis. From a diagnostic point of view, a single autoantigen based diagnosis can be improved by increasing sensitivity and specificity by using a panel of markers where multiple auto-antibodies are being detected simultaneously. Particular preferred combinations are of markers within one of the marker lists 2 to 31 as identified further herein.

The markers are suitable protein antigens that are overexpressed in tumor and can be used to either identify cancerous tissue or to differentiate between specific stages of cancer and pre-cancerous development, or both. The markers usually cause an antibody reaction in a patient. Therefore the most convenient method to detect the presence of these markers in a patient is to detect antibodies against these marker proteins in a sample from the patient, especially a body fluid sample, such as blood or serum.

To detect an antibody in a sample it is possible to use marker proteins as binding agents and subsequently to detect bound antibodies. It is not necessary to use the entire marker proteins but it is sufficient to use antigenic fragments that are bound by the antibodies. "Antigenic fragment" herein relates to a fragment of the marker protein that causes an immune reaction against said marker protein in a human. Preferred antigenic fragments of any one of the marker proteins are the fragments of the clones as identified by the UniqueID. Such antigenic fragments may be antigenic in a plurality of humans, such as at least 5, or at least 10 individuals.

"Diagnosis" for the purposes of this invention means the positive determination of colon carcinoma by means of the marker proteins according to the invention as well as the assignment of the patients to colon carcinoma. The term "diagnosis" covers medical diagnostics and examinations in this regard, in particular in-vitro diagnostics and laboratory diagnostics, likewise proteomics and peptide blotting. Further tests can be necessary to be sure and to exclude other diseases. The term "diagnosis" therefore likewise covers the differential diagnosis of colon carcinoma by means of the marker proteins according to the invention and the risk or prognosis of colon carcinoma.

Specific indications that can be identified with one or more of the markers are cancer and pre-cancerous polyps, in particular also the differentiation between high-risk and low-risk polyps. Particular differentiations that can be made with the inventive markers and methods are distinguishing 1) healthy conditions vs. cancer plus pre-cancerous polyps (both high-risk and low-risk) 2) healthy conditions vs. cancer, 3) healthy conditions plus low-risk polyps vs. high-risk polyps plus cancer, 4) cancer vs. high-risk polyps vs. low-risk polyps vs. healthy conditions, 5) healthy conditions vs. low-risk polyps, 6) low-risk polyps vs. high-risk polyps and 7) high-risk polyps vs. cancer.

The invention can be used to distinguish 2 or more indications. If more than 2 indications, e.g. 3 or 4 such as in indication group 4) mentioned above, are distinguished it is preferred to use stepwise statistical analysis to distinguish the individual conditions. In preferred embodiments, in such a stepwise analysis, in a first step one indication is distinguished from the remaining indications, e.g. healthy conditions vs. the combined group of cancer, high-risk polyps and low-risk polyps. Stepwise, one additional indication is removed from the remaining group and distinguished from the next remaining indications. E.g. for group 4) it is preferred to distinguish in the second step low-risk polyps vs. the group of cancer and high-risk polyps, and consequently in the third step to distinguish cancer vs. high-risk polyps (see e.g. example 11). Such a statistical method is e.g. the binary tree analysis.

According to the invention, adenomatous villous, adenomatous tubulovillous, and co-occurrence of adenomatous tubular with tubulovillous polyps were classified as high-risk polyps while hyperplastic polyps and adenomatous tubular polyps were assigned to the low-risk group. The presence of high-risk polyps (and the presence of high-risk polyp markers) is an indication for a surgical intervention to remove the polyp since a rapid development to full cancer is likely. Contrary thereto, low-risk polyps do not require immediate surgical intervention but it is advised to further monitor the patients for the occurrence of high-risk polyp or cancer markers.

Disclosed is also a surgical method comprising detecting cancer or a high-risk polyp according to the present invention and removing said cancer or high-risk polyp. Of course, to be on the safe side it is also possible to similarly detect a low-risk polyp and remove said low-risk polyp.

In preferred methods of the invention the diagnosis of a risk for cancer comprises detecting polyps, in particular high-risk polyps and/or low-risk polyps, in a patient.

In particular the inventive method according to the claims may comprise distinguishing between combined groups of cancer and pre-cancerous states, selected from distinguishing healthy conditions vs. cancer plus pre-cancerous polyps (both high-risk and low-risk), healthy conditions vs. cancer, healthy conditions plus low-risk polyps vs. high-risk polyps, healthy conditions plus low-risk polyps vs. high-risk polyps plus cancer, healthy conditions vs. low-risk polyps, low-risk polyps vs. high-risk polyps, and high-risk polyps vs. cancer. A positive result in distinguishing healthy conditions plus low-risk polyps vs. high-risk polyps plus cancer can prompt a further cancer test, in particular more invasive tests than a blood test such as an endoscopy or a biopsy.

The disclosed markers are preferably grouped in sets of high distinctive value. Some sets excel at diagnosing or distinguishing 1, 2, 3, 4, 5, 6 or 7 of the above identified indications.

Preferred markers are of List 2, which comprise markers for all of the above indications 1) to 7).
**List 2:** Preferred marker protein set, suitable for multiple analytic distinctions; Proteins are identified by protein symbol: ACAA2, ACTL6B, ARHGEF1, ARHGEF10L, ASB13, ATXN2, C10orf76, C17orf28, TMEM98, C17orf90, AFP, AFP, COL3A1, CUL1, DCTPP1, DEF8, EIF4A2, RPA1, TACC2, ACTL6B, PLEKHO1, HAUS4, BTBD6, ISG15, LRRC4C, LTBP3, MACF1, MTCH2, NARFL, NBEAL2, KPNA2, PAPLN, PDIA3, PDP1, PI4KA, PKM2, PLAA, PLCG1, PLXND1, RNF40, RPL37A, SERPINA1, SLC4A3, SPRY1, TMCC2, TSTA3, UROD, PAICS, VPS18, ZNF410, ZNF668.

Disclosed is a method of diagnosing colon cancer or the risk of colon cancer in a patient by detecting at least 2 of the marker proteins selected from the markers of List 2 in a patient comprising the step of detecting antibodies binding said marker proteins, detecting said marker proteins or antigenic fragments thereof in a sample of the patient. Also disclosed is a method of diagnosing colon cancer or the risk of colon cancer in a patient by detecting at least 20%, preferably at least 30%, especially preferred at least 40%, at least 50%, at least 60%, at least 70%, at least 80% at least 90% or all of the marker proteins selected from the markers of List 2 in a patient comprising the step of detecting antibodies binding said marker proteins, detecting said marker proteins or antigenic fragments thereof in a sample of the patient.

Further disclosed marker sets are provided in example 7 as lists 3 to 31. Thus the present disclosure also provides the method of diagnosing colon cancer or the risk of colon cancer in a patient by detecting at least 2 of the marker proteins selected from the markers of List 3, 4, 5, 6, 7, 8, 9, 10, 11, 12, 13, 14, 15, 16, 17, 18, 19, 20, 21, 22, 23, 24, 25, 26, 27, 28, 29, 30, 31 or any combination thereof in a patient comprising the step of detecting antibodies binding said marker proteins, detecting said marker proteins or antigenic fragments thereof in a sample of the patient. Further disclosed is a method of diagnosing colon cancer or the risk of colon cancer in a patient by detecting at least 20%, preferably at least 30%, especially preferred at least 40%, at least 50%, at least 60%, at least 70%, at least 80% at least 90% or all, of the marker proteins selected from the markers of List 3, 4, 5, 6, 7, 8, 9, 10, 11, 12, 13, 14, 15, 16, 17, 18, 19, 20, 21, 22, 23, 24, 25, 26, 27, 28, 29, 30, 31 or any combination thereof in a patient comprising the step of detecting antibodies binding said marker proteins, detecting said marker proteins or antigenic fragments thereof in a sample of the patient.

Also disclosed is a method of diagnosing colon cancer or the risk of colon cancer in a patient by detecting a marker protein selected from any one of List 1 in a patient comprising the step of detecting antibodies binding said marker protein, detecting said marker protein or antigenic fragments thereof in a sample of the patient. Of course, preferably more than one marker protein is detected. As noted with regards to the marker combinations of sets of lists 2 to 31, preferably at least 2, but also 3, 4, 5, 6, 7, 8, 9, 10, 11, 12, 13, 14, 15, 16, 17, 18, 19, 20, 21, 22, 23, 24, or 25 or more, of the marker proteins can be detected. This relates to any one of the sets of lists 1 to 31. Even more preferred at least 20%, at least 30%, at least 40%, at least 50%, at least 60%, at least 70%, at least 80%, at least 90%, at least 95% or all of the markers of any set of any of the lists 1 to 31 are used in a diagnostic set. Such parts of at least 2 markers or at least 20% markers (or more as indicated) are also referred to as subsets herein.

Such a marker combination of a particular list or any combination of marker selection thereof are referred to herein as diagnostic set. Such sets constitute a further aspect of the disclosure and kits are provided comprising diagnostic agents (such as binding moieties) to detect such markers. The entire disclosure herein relates to both the inventive uses of kits according to the claims as well as the methods themselves, that make use of agents that can be comprised in the kit.

Preferred combinations are of markers that are particularly indicative for a specific distinction as given in table 1 below.

Preferred marker combinations are of 2, 3, or 4 lists selected from lists 3, 4, 19 and 20. These lists, as well as any combination are particularly effective for distinguishing indication 1, healthy conditions vs. cancer plus pre-cancerous polyps (both high-risk and low-risk) and are preferably used therefore. Of course, not all of the markers are usually necessary since subsets also have sufficient diagnostic power. Preferably at least 2 markers or at least 20% of the markers (or any higher number as given above) of these lists or combined lists are used in the methods.

Preferred marker combinations are of 2, 3, 4, 5 or 6 lists selected from lists 5, 6, 7, 8, 21 and 24. These lists, as well as any combination are particularly effective for distinguishing indication 2 healthy conditions vs. cancer and are preferably used therefore. Of course, not all of the markers are usually necessary since subsets also have sufficient diagnostic power. Preferably at least 2 markers or at least 20% of the markers (or any higher number as given above) of these lists or combined lists are used in the methods.

Preferred marker combinations are of 2, 3, 4 or 5 lists selected from lists 9, 10, 11, 25 and 26. These lists, as well as any combination are particularly effective for distinguishing indication 3, healthy conditions plus low-risk polyps vs. high-risk polyps plus cancer, and are preferably used therefore. Of course, not all of the markers are usually necessary since subsets also have sufficient diagnostic power. Preferably at least 2 markers or at least 20% of the markers (or any higher number as given above) of these lists or combined lists are used in the methods.

Preferred marker combinations are of 2 or 3 lists selected from lists 12, 13 and 27. These lists, as well as any combination are particularly effective for distinguishing indication 4 cancer vs. high-risk polyps vs. low-risk polyps vs. healthy conditions, and are preferably used therefore. Of course, not all of the markers are usually necessary since subsets also have sufficient diagnostic power. Preferably at least 2 markers or at least 20% of the markers (or any higher number as given above) of these lists or combined lists are used in the methods.

A preferred marker combination is of the 2 lists selected from lists 14 and 28. These lists, as well as any combination are particularly effective for distinguishing indication 5, healthy conditions vs. low-risk polyps, and are preferably used therefore. Of course, not all of the markers are usually necessary since subsets also have sufficient diagnostic power. Preferably at least 2 markers or at least 20% of the markers (or any higher number as given above) of these lists or combined lists are used in the methods.

Preferred marker combinations are of 2 or 3 lists selected from lists 15, 16 and 29. These lists, as well as any combination are particularly effective for distinguishing indication 6, low-risk polyps vs. high-risk polyps, and are preferably used therefore. Of course, not all of the markers are usually necessary since subsets also have sufficient diagnostic power. Preferably at least 2 markers or at least 20% of the markers (or any higher number as given above) of these lists or combined lists are used in the methods.

Preferred marker combinations are of 2, 3 or 4 lists selected from lists 17, 18, 30 and 31. These lists, as well as any combination are particularly effective for distinguishing indication 7, high-risk polyps vs. cancer, and are preferably used therefore. Of course, not all of the markers are usually necessary since subsets also have sufficient diagnostic power. Preferably at least 2 markers or at least 20% of the markers (or any higher number as given above) of these lists or combined lists are used in the methods.

In especially preferred embodiments, the combination is of lists 4 and 20, wherein the markers are selected from ACADVL, ADH5, AGFG1, ALDOA, ARHGAP21, ARHGEF1, ASB13, ATXN10, BCKDHA, BCS1L, BIN3, C10orf76, C17orf28, TMEM98, C17orf90, AFP, AFP, CCNI, CDK16, CHD8, COL3A1, CPLX1, CPNE6, CTDP1, CYC1, DCTPP1, DEF8, EID1, EIF4A2, RPA1, TACC2, ACTL6B, FAM160A2, HMGN2, FASN, C10orf2, HAUS4, JMJD4, BTBD6, IMP4, LAMB3, LCP1, LMO7, LMTK2, LRRC4C, LTBP3, MRPL47, MRPS11, MTCH2, MTSS1L, NARFL, NBEAL2, NME2, NRBP2, NOA1, PAPLN, PDIA3, PDP1, PI4KA, PIK3R2, PKM2, PLAA, PLCG1, PLXND1, PPP1R2, PPP4R1, PSMA2, PSMC2, RAD1, RBM4, RBM4, RPL26, RPL37A, SBF2, SERPINA1, SF3B4, SLC4A3, SLC25A29, SNRNP40, SPRY1, SSRP1, MGAT4B, TMUB2, ST3GAL3, TSTA3, UBE2L3, UMPS, UROD, USP7, PAICS, VASP, VPS18, VPS72, WDR13, YARS, ZNF410, ZNF668. These markers as well as the combined set of any one of at least 2 markers or at least 20% of said markers (or any higher number as indicated above) is particularly suitable for distinguishing healthy conditions vs. cancer plus pre-cancerous polyps (both high-risk and low-risk) and is preferably used for this diagnosis.

In especially preferred embodiments, the combination is of lists 5, 6, 7, 21, and 24 and wherein the markers are selected from A1BG, ACAA2, ACTL6B, ACTR1B, ADCK3, AFTPH, AKAP9, AP3D1, APBA3, ARAP1, CHST10, ARHGEF1, ARHGEF10L, ARHGEF17, ASB13, ASNA1, ATP5H, ATXN2, BCL6, C11orf80, TMEM98, C17orf90, AFP, AFP, C18orf32, C6orf136, CAMK1D, CCL28, CCND1, CDH2, CEP57, CKM, COL3A1, COL6A3, COL8A2, HSPA1A/HSPA1B, CPLX1, CSK, CSRNP1, CTDP1, CTNND2, CUL1, CUL9, CYC1, DCAF15, DCTPP1, DDX19B, DDX3Y, DDX54, DEF8, DENND5A, DNAJC10, DOCK10, DOCK9, DPYSL5, EEF1A1, EHMT2, EIF3L, EIF4A2, EML2, ERBB3, TACC2, ACTL6B, PLEKHO1, EXOSC8, FAM204A, FBN1, GEMIN2, GGA1, GP1BB, GPSM1, GSTP1, GTF3A, HADHA, HAUS4, HK1, HMG20B, HMGN2, HMGN2, HNRNPK, BTBD6, ICAM3, IFNGR2, IMP4, INTS7, ISG15, IVNS1ABP, JMJD7-PLA2G4B, KIAA0368, KIF3C, LIMD2, LPCAT1, LRRC4C, LRSAM1, MACF1, MAN2B1, MCM3AP, METTL2B, MTA1, MTCH2, MTSS1L, NAGLU, NARFL, NASP, NBEAL2, NCAN, NFKB2, NHEJ1, NME2, KPNA2, NPDC1, OTUD5, PAPLN, PBRM1, PBX2, PDP1, PDZD4, PHACTR3, LOC440354, PKD1L1, PKM2, PLAA, PLCG1, PLEKHA5, PLXNA1, PLXND1, PMVK, POGZ, POLN, POMGNT1, POTEE/POTEF, PPCDC, PPP4R1, PRDM2, PRKACA, PRKCSH, PRMT1, TSTA3, PRPF4B, PSMB4, PSMB6, QTRT1, RAD1, RBPJ, REV3L, RNF220, RPL24, RPL37A, RPL7, RPS7, RSL1D1, RSL1D1, RTN4, RUVBL2, SAFB2, SAMSN1, GRK6, SERBP1, SERPINA1, SETD1B, SGSH, SLC4A3, SLMAP, SNTA1, SPRY1, ST6GALNAC1, STAB1, SUOX, TAGLN3, TIAL1, TMC8, TMCO7, TNFAIP2, TPX2, TRAPPC3, ST3GAL3, TRIOBP, TRIP12, TRPC4AP, TSTA3, TTR, TTYH3, TUBB3, TUBGCP2, UBB, UMPS, UROD, UROD, PAICS, VPS18, YLPM1, YWHAZ, ZCCHC14, ZNF174, ZNF410, ZNF589. These markers as well as the combined set of any one of at least 2 markers or at least 20% of said markers (or any higher number as indicated above) is particularly suitable for distinguishing healthy conditions vs. cancer and is preferably used for this diagnosis.

In especially preferred embodiments, the combination is of lists 9-11 and 25-26 and wherein the markers are selected from ABCE1, ACAA2, ACTL6B, ADH5, AKT2, ANKRD36B, ANXA6, APBB1IP, ARHGAP21, ARHGEF10L, ARHGEF25, ASAP1, ASB13, ASPSCR1, AXIN1, BCKDHA, C10orf76, TMEM98, C17orf90, AFP, AFP, C18orf32, PECAM1, C6orf136, CCL28, IK, CDK16, CDT1, CERCAM, CHCHD8, CLDN5, CLU, COMMD9, COX7A2L, CPE, CPNE2, DBI, DCAF11, DCTPP1, DDR1, DDX27, DENR, DHX36, DHX58, DLG5, DTNBP1, EID1, EIF4A2, EPC1, TACC2, ACTL6B, PLEKHO1, EXOSC4, FAM213A, FBLL1, FBRS, FKBP15, FLII, FN1, GNAO1, GOSR1, GRK6, GTF2B, HAPLN3, HARS, HAUS4, HDAC6, HEBP2, HLA-C, JMJD4, HNRNPA2B1, HNRNPK, HNRNPR, HNRPLL, ELAC2, HSPA8, INPP5K, INPPL1, INTS1, INTS1, ISG15, KDM4A, LAMB1, LCP1, LOC100132116, LRIG1, LRIG1, LRRC16B, LRRC4C, LTBP3, LYSMD2, MAF1, MRPL38, MTCH1, MTCH2, RPL17, MYO1C, MYO5A, NARF, NARFL, NBEAL2, NBR1, NDUFB10, NME2, KPNA2, OTUD1, PABPC1, PAPLN, PDIA3, LOC440354, PI4KA, PIK3CD, PIK3IP1, PJA1, PKD1L1, PKM2, PLAA, PKP3, PLXNA3, PLXND1, POMT1, PPP1R13B, PPP4R1, PPP5C, PRDX5, PRRT1, PRRT2, PSKH1, PSMB4, REC8, RGS19, RHBDD2, RPL4, RNF40, RPL13, RPL17, RPL22, RPL24, RPL37A, RPN1, SAT2, SERPINA1, SERPINH1, SGSH, SH3BP2, SLC25A20, SMCHD1, SNTA1, SPRY1, SRRM2, STMN4, TBC1D9B, TBCB, TIAL1, TIAM2, TMC8, TMCC2, TPM3, ZWINT, TRAPPC3, TRAPPC4, TSTA3, TTYH1, GPX4, TUBB3, UBC, UBE2N, USP48, VARS, PAICS, VPS18, YLPM1, YPEL1, ZCCHC11, ZFP14, ZNF133, ZNF232, ZNF410, ZNF668, ZNF672. These markers as well as the combined set of any one of at least 2 markers or at least 20% of said markers (or any higher number as indicated above) is particularly suitable for distinguishing healthy conditions plus low-risk polyps vs. high-risk polyps plus cancer and is preferably used for this diagnosis.

In especially preferred embodiments, the combination is of lists 14 and 28 and wherein the markers are selected from AHSG, AKR1B1, AP1B1, ARPP21, ATP2A3, BMS1P5, BTBD11, CANX, CCL28, CDK16, CPNE1, DCTPP1, DDX19A, DHX8, EHD4, RPA1, ELAVL1, TACC2, FBXO21, C10orf2, HMGCL, IKBKAP, ITGAL, LMTK2, LRPAP1, MAGED1, MAPKAP1, MARS, MAST2, MATR3, MUC2, MYO5A, NAV2, NDUFS2, NEFL, PDP1, PEX19, PFKL, PKM2, PLAA, PLEKHM2, MED4 (includes EG:29079), PNCK, PPP2R5C, PSMD6, PTPRS, BAD, RNF10, RPL17, RPL37A, ACO2 (includes EG:11429), SLC4A3, STK17A, SUV420H1, TBC1D7, TBC1D9B, TRMT2A, TUBA1B, TUBB6, WDR1, WDR5, ZNF277, ZNF514. These markers as well as the combined set of any one of at least 2 markers or at least 20% of said markers (or any higher number as indicated above) is particularly suitable for distinguishing healthy conditions vs. low-risk polyps and are preferably used for this diagnosis.

Some markers are more preferred than others. Especially preferred markers are those which are represented at least 5, at least 6, at least 7, at least 8, at least 9, at least 10, at least 11, at least 12 times in any one of lists 3 to 31. These markers are preferably used in any one of the methods or sets.

Less preferred markers are selected from AGBL5, AKT1, AKT2, C21orf2, C6orf192, C9orf43, COASY, EFNA3, EPRS, FAS, GOLGA4, GOLGB1, HDAC5, HIP1R, HSPA4, KIAA1416, CHD7, KIF2C, LMNA, MAP-KAPK3, MBD2, NFYA, NHSL1, NUCB1, NY-CO-16, RPS6KA1, RPS6KA2, SDCCAG1, SDCCAG3, SREBF2, STAU1, STUB1, TAX1BP1, TRIP4, TTLL7, WBP2, Znf292, BHMT2, BMX, Cbx5, CEA, C-myc, CSNK1G2, CTAG1A, DAPK1, FGFR4, GRK7, HDAC1, Her-2/neu, HMGN2, HMMR, HSPH1, IGLC1, Imp1, IRAK4, ITGA6, KDR, Koc, LGR6, LIMS1, LMTK2, MAGEA3, MAP-KAPK5, MFAP2, MKNK1, NAP1L1, NEK3, NMDAR, NOXA1, NY-CO-41, NY-CO-8, NY-ESO-1, P62, PBK, PDE4A, PDGFRB, PDXK, PFDN5, PIM1 , PKN1, PKN2, PRKCD, RBMS1, RBPJ, RIOK1, SALL2, SCP2, SDCCAG10, SDCCAG8, Seb4D, SRC, SSRP1, SSX2, STARD10, STK4, TAF10, TDRD6, TP53, TPM4, TRIM21, TSHZ1, TSLP, UBE3A, USH1C, ZNF706. Preferably none of these markers is used in the inventive methods or present in one of the disclosed set. As an alternative to this exclusion, these less preferred markers may just not be a requirement of any of the disclosed sets, i.e. the sets or lists herein may be read as if any one of these less preferred markers is not a recited therein.

The present disclosure also relates to a method of selecting such at least 2 markers (or more as given above) or at least 20 % of the markers (or more as given above) of any one of the inventive sets with high specificity. Such a method includes comparisons of signal data for the inventive markers of any one of the inventive markers sets, especially as listed in lists 1 to 31, with said signal data being obtained from controls samples of known conditions or indications and further statistically comparing said signal data with said conditions thereby obtaining a significant pattern of signal data capable of distinguishing the conditions of the known control samples.

In particular, the controls may comprise one or more cancerous control (preferably at least 5, or at least 10 cancerous controls) and/or a pre-cancerous polyp (e.g. high risk or low risk polyps) control (preferably at least 5, or at least 10 pre-cancerous controls) and/or a healthy control (preferably at least 5, or at least 10 healthy controls). Preferably at least 2 different indications are selected that shall be distinguished. In preferred embodiments, the control comprises samples for the indications selected from indications 1), 2), 3), 4), 5), 6), and 7) as mentioned above.

The controls can be used to obtain a marker dependent signal pattern as indication classifier. Such a signal pattern can be obtained by routine statistical methods, such as binary tree methods. Common statistical methods calculate a (optionally multi-dimensional) vector within the multitude of control data signal values as diagnostically significant distinguishing parameter that can be used to distinguish one or more indications from other one or more indications. The step usually comprises the step of "training" a computer software with said control data. Such pre-obtained training data or signal data can be provided on a computer-readable medium to a practitioner who performs the diagnosis.

Preferably, the method comprises optimizing the selection process, e.g. by selecting alternative or additional markers and repeating said comparison with the controls signals, until a specificity and/or sensitivity of at least 75% is obtained, preferably of at least 80%, at least 85%, at least 90%, at least 95%.

Thus as mentioned, the present disclosure also relates to diagnosing cancer or pre-cancerous polyps, preferably wherein said precancerous polyps are distinguished between high-risk polyps and low-risk polyps, wherein said high-risk polyps comprise adenomatous villous, adenomatous tubulovillous, or co-occurrence of adenomatous tubular with tubulovillous polyps. For any one of the particular distinctions, preferably at least one of the markers or marker lists or subsets thereof, which contains said indication selected from cancer, high-risk polyps, low risk polyps as given above and in table 1 is used.

"Marker" or "marker proteins" are diagnostic indicators found in a patient and are detected, directly or indirectly by the inventive methods. Indirect detection is preferred. In particular, all of the disclosed markers have been shown tc cause the production of (auto)antigens in cancer patients or patients with a risk of developing cancer. The easiest way to detect these markers is thus to detect these (auto)antibodies in a blood or serum sample from the patient. Such antibodies can be detected by binding to their respective antigen in an assay. Such antigens are in particular the marker proteins themselves or antigenic fragments thereof. Suitable methods exist in the art to specifically detect such antibody-antigen reactions and can be used according to the invention. Preferably the entire antibody content of the sample is normalized (e.g. diluted to a preset concentration) and applied to the antigens. Preferably the IgG, IgM, IgD, IgA or IgE antibody fraction, is exclusively used. Preferred antibodies are IgG. Preferably the subject is a human.

Binding events can be detected as known in the art, e.g. by using labeled secondary antibodies. Such labels can be enzymatic, fluorescent, radioactive or a nucleic acid sequence tag. Such labels can also be provided on the binding means, e.g. the antigens as described in the previous paragraph. Nucleic acid sequence tags are especially preferred labels since they can be used as sequence code that not only leads to quantitative information but also to a qualitative identification of the detection means (e.g. antibody with certain specificity). Nucleic acid sequence tags can be used in known methods such as Immuno-PCR. In multiplex assays, usually qualitative information is tied to a specific location, e.g. spot on a microarray. With qualitative information provided in the label, it is not necessary to use such localized immunoassays. In is possible to perform the binding reaction of the analyte and the detection means, e.g. the serum antibody and the labeled antigen, independent of any solid supports in solution and obtain the sequence information of the detection means bound to its analyte. A binding reaction allows amplification of the nucleic acid label in a detection reaction, followed by determination of the nucleic acid sequence determination. With said determined sequence the type of detection means can be determined and hence the marker (analyte, e.g. serum antibody with tumor associated antigen specificity).

In preferred embodiments of the invention the step of detecting antibodies binding said marker proteins, detecting said marker proteins or antigenic fragments thereof comprises comparing said detection signal with detection signals of a healthy control and comparing said detection signals, wherein an increase in the detection signal indicates colon cancer or said risk of colon cancer.

In preferred embodiments of the invention the step of detecting antibodies binding said marker proteins, detecting said marker proteins or antigenic fragments thereof comprises comparing said detection signal with detection signals of a cancerous control or a pre-cancerous polyp (e.g. high risk or low risk polyps) control and comparing said detection signals. Alternatively or in addition, the comparison can also be with a healthy control, a control of a low-risk polyp, a control with a high risk polyp or any combination thereof. In preferred embodiments, the control comprises the indications that are intended to be distinguished, such as indications 1), 2), 3), 4), 5), 6), and 7) as mentioned above. In particular preferred, especially in cases of using more marker sets of 2 or more markers as mentioned above, a statistical analysis of the control is performed, wherein the controls are used to obtain a marker dependent signal pattern as indication classifier and the marker dependent signals of the sample to be analysed is compared with and/or fitted onto said pattern thereby obtaining information of the diagnosed condition or indication. Such a signal pattern can be obtained by routine statistical methods, such as binary tree methods. Common statistical methods calculate a (optionally multi-dimensional) vector within the multitude of control data signal values as diagnostically significant distinguishing parameter that can be used to distinguish one or more indications from other one or more indications. Such statistical analysis is usually dependent on the used analytical platform that was used to obtain the signal data, given that signal data may vary from platform to platform. Such platforms are e.g. different microarray or solution based setups (with different labels or analytes - such as antigen fragments - for a particular marker). Thus the statistical method can be used to calibrate each platform to obtain diagnostic information with high sensitivity and specificity. The step usually comprises the step of "training" a computer software with said control data. Alternatively, pre-obtained training data can be used. Such pre-obtained training data or signal data can be provided on a computer-readable medium to a practitioner.

In further embodiments a detection signal from the sample of a patient in amplitude of at least 60%, preferably at least 80%, of the cancerous control indicates colon cancer or said risk of colon cancer.

Usually not all of the markers or detection agents may lead to a signal. Nevertheless only a fraction of the signals is suitable to arrive at a diagnostic decision. In preferred embodiments of the invention a detection signal in at least 60%, preferably at least 70%, least 75%, at least 85%, or in particular preferred at least 95%, even more preferred all, of the used markers indicates colon cancer or said risk of colon cancer.

The present diagnostic methods further provide necessary therapeutic information to decide on a surgical intervention. Therefore the present disclosure also provides a method of treating a patient comprising colon cancer or having a polyp with a high risk of developing colon cancer, comprising detecting cancer or a polyp with a high risk of developing colon cancer according to any aspect or embodiment of the disclosure and removing said colon cancer or polyp. "Stratification or therapy control" for the purposes of this disclosure means that the method according to the disclosure renders possible decisions for the treatment and therapy of the patient, whether it is the hospitalization of the patient, the use, effect and/or dosage of one or more drugs, a therapeutic measure or the monitoring of a course of the disease and the course of therapy or etiology or classification of a disease, e.g., into a new or existing subtype or the differentiation of diseases and the patients thereof. In a further embodiment of the disclosure, the term "stratification" covers in particular the risk stratification with the prognosis of an outcome of a negative health event.

One skilled in the art is familiar with expression libraries, they can be produced according to standard works, such as Sambrook et al, "Molecular Cloning, A laboratory handbook, 2nd edition (1989), CSH press, Cold Spring Harbor, N.Y. Expression libraries are also preferred which are tissue-specific (e.g., human tissue, in particular human organs). Members of such libraries can be used as antigen for use as detection agent to bind analyte antibodies. Furthermore included are expression libraries that can be obtained by exon-trapping. A synonym for expression library is expression bank. Also preferred are protein biochips or corresponding expression libraries that do not exhibit any redundancy (so-called: Uniclone(R) library) and that may be produced, for example, according to the teachings of WO 99/57311 and WO 99/57312. These preferred Uniclone libraries have a high portion of nondefective fully expressed proteins of a cDNA expression library. Within the context of this invention, the antigens can be obtained from organisms that can also be, but need not be limited to, transformed bacteria, recombinant phages, or transformed cells from mammals, insects, fungi, yeasts, or plants. The marker antigens can be fixed, spotted, or immobilized on a solid support. Alternatively, is is also possible to perform an assay in solution, such as an Immuno-PCR assay.

In a further aspect, the present invention provides a use of a kit suitable to detect autoantibodies against any marker combination, wherein said diagnostic agents comprise marker proteins selected from ArsA Arsenite Transporter, ATP-Binding, Homolog 1 (ASNA1) and at least 1 of the marker proteins selected from the markers Ecm29 proteasome adaptor and scaffold (KIAA0368 also known as ECPAS), queuine tRNA-ribosyltransferase catalytic subunit 1 (QTRT1), SAM domain, SH3 domain and nuclear localization sig-nals 1 (SAMSN1), glycoprotein Ib platelet subunit beta (GP1BB), actin like 6B (ACTL6B), aftiphilin (AFTPH), A-kinase anchoring protein 9 (AKAP9), Rho guanine nucleotide exchange factor 1 (ARHGEF1), C-C motif chemokine ligand 28 (CCL28), cadherin 2 (CDH2), C-terminal Src kinase (CSK), dCTP pyrophos-phatase 1 (DCTPP1), DEAD-box helicase 3 Y-linked (DDX3Y), DnaJ heat shock protein family (Hsp40) member C10 (DNAJC10), EMAP like 2 (EML2), golgi associated, gamma adaptin ear containing, ARF binding protein 1 (GGA1), hydroxyacyl-CoA dehydrogenase trifunctional multienzyme complex subunit alpha (HADHA), high mobility group nucleosomal binding domain 2 (HMGN2), ISG15 ubiquitin like modifier (ISG15), leucine rich repeat and ster-ile alpha motif containing 1 (LRSAM1), minichromosome mainte-nance complex component 3 associated protein (MCM3AP), mito-chondrial carrier 2 (MTCH2), karyopherin subunit alpha 2 (KPNA2), polycystin 1 like 1, transient receptor potential channel interacting (PKD1L1), pogo transposable element de-rived with ZNF domain (POGZ), protein arginine methyltransfer-ase 1 (PRMT1), pre-mRNA processing factor 4B (PRPF4B), G pro-tein-coupled receptor kinase 6 (GRK6), SERPINE1 mRNA binding protein 1 (SERBP1), trafficking protein particle complex 3 (TRAPPC3), ST3 beta-galactoside alpha-2,3-sialyltransferase 3 (ST3GAL3), YLP motif containing 1 (YLPM1), tyrosine 3-monooxygenase/tryptophan 5-monooxygenase activation protein zeta (YWHAZ) or antigenic fragments thereof suitable to bind the autoantibodies in a sample; in a method of the invention. Disclosed is a kit of diagnostic agents suitable to detect any marker or marker combination as described above, preferably wherein said diagnostic agents comprise marker proteins or antigenic fragments thereof suitable to bind antibodies in a sample, especially preferred wherein said diagnostic agents are immobilized on a solid support or in solution, especially when said markers are each labelled with a unique label, such as a unique nucleic acid sequence tag. The kit may further comprise detection agents, such as secondary antibodies, in particular anti-human antibodies, and optionally also buffers and dilution reagents. The disclosure therefore likewise relates to the object of providing a diagnostic device or an assay, in particular a protein biochip, ELISA or Immuno-PCR assay, which permits a diagnosis or examination for colon carcinoma.

Additionally, the marker proteins (as binding moieties for antibody detection) can be present in the respective form of a fusion protein, which contains, for example, at least one affinity epitope or tag. The tag may be one such as contains c-myc, his tag, arg tag, FLAG, alkaline phosphatase, VS tag, T7 tag or strep tag, HAT tag, NusA, S tag, SBP tag, thioredoxin, DsbA, a fusion protein, preferably a cellulose-binding domain, green fluorescent protein, maltose-binding protein, calmodulin-binding protein, glutathione S-transferase, or lacZ, a nanoparticle or a nucleic acid sequence tag. Such a nucleic acid sequence can be e.g. DNA or RNA, preferably DNA.

In all of the embodiments, the term "solid support" covers embodiments such as a filter, a membrane, a magnetic or fluorophore-labeled bead, a silica wafer, glass, metal, ceramics, plastics, a chip, a target for mass spectrometry, or a matrix. However, a filter is preferred according to the invention.

As a filter, furthermore PVDF, nitrocellulose, or nylon is preferred (e.g., Immobilon P Millipore, Protran Whatman, Hybond N+ Amersham).

In another preferred embodiment the arrangement corresponds to a grid with the dimensions of a microtiter plate (8-12 wells strips, 96 wells, 384 wells, or more), a silica wafer, a chip, a target for mass spectrometry, or a matrix.

Preferably the kit also comprises non-diagnostic control proteins, which can be used for signal normalization. These control proteins bind to moieties, e.g. proteins or antibodies, in the sample of a diseased patient same as in a healthy control. In addition to the marker proteins any number, but preferably at least 2 controls can be used in the method or in the kit.

Preferably the kit is limited to a particular size. According to these embodiments the kit comprises at most 3000 diagnostic agents, preferably at most 2500 diagnostic agents, at most 2000 diagnostic agents, at most 1500 diagnostic agents, at most 1200 diagnostic agents, at most 1000 diagnostic agents, at most 800 diagnostic agents, at most 500 diagnostic agents, at most 300 diagnostic agents, at most 200 diagnostic agents, at most 100 diagnostic agents, such as marker proteins or antigenic fragments thereof.

In especially preferred embodiments the kit further comprises a computer-readable medium or a computer program product, such as a computer readable memory devices like a flash storage, CD-, DVD- or BR-disc or a hard drive, comprising signal data for the control samples with known conditions selected from cancer or a pre-cancerous polyp (such as high risk polyps and/or low risk polyps) and/or of healthy controls, and/or calibration or training data for analysing said markers provided in the kit for diagnosing colon cancer or distinguishing conditions or indications selected from healthy conditions, cancer, high-risk polyps and low-risk polyps. Especially preferred are the indications 1), 2), 3), 4), 5), 6) and 7) mentioned above.

The kit may also comprise normalization standards, that result in a signal independent of a healthy condition and cancerous or pre-cancerous condition. Such normalization standards can be used to obtain background signals. Such standards may be specific for ubiquitous antibodies found in a human, such as antibodies against common bacteria such as *E. coli.* Preferably the normalization standards include positive and negative (leading to no specific signal) normalization standards.

The present invention is further illustrated by the following figures and examples, without being limited to these embodiments of the invention.

### Figures:

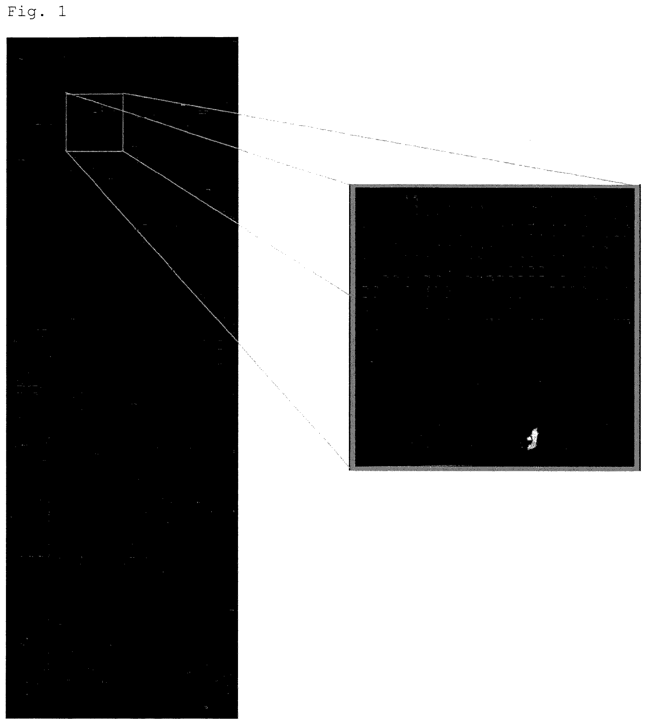

**Fig. 1****:** Example of a scanned 16k protein-microarray. In the detail right side, subarray 6 is depicted.

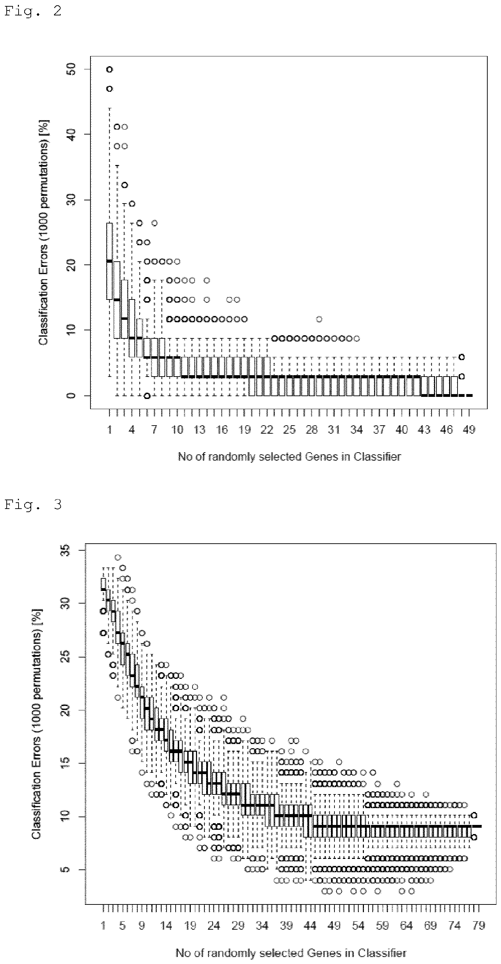

**Fig. 2****:** Subsets of classifier markers form the panels defined by the 50 classifier markers for distinguishing Cancer vs. Controls were selected and randomly chosen subsets of 2-49 markers (x-axis) were selected 1000-times. The classification success obtained by that subset using the nearest centroid classifier algorithm is shown on the y-axis.
**Fig. 3****:** The 80 classifier markers for distinguishing Disease (Cancer & polyps) vs. Controls were selected and randomly chosen subsets of 2-79 markers (x-axis) were selected 1000-times. The classification success obtained by that subset using the nearest centroid classifier algorithm is shown on the y-axis.

### Examples:

### Example 1: Patient samples

Biomarker screening has been performed with serum samples from a test set of serum samples derived from 49 individuals with confirmed colon-carcinoma, 50 healthy controls, 17 patients with high-risk polyps, and 18 patients with low-risk polyps (n=134). All these individuals have been elucidated by a positive FOBT test and underwent colonoscopy. The differentiation of carcinoma (denoted *Carc*)*,* high and low risk polyps (denoted *HR* and *LR*, respectively) and controls (denoted *Contr*) was conducted during clinical examination of patients and tissue samples.

### Example 2: Immunoglobuline (IgG) purification from the serum or plasma samples

The patient serum or plasma samples were stored at -80 °C before they were put on ice to thaw them for IgG purification using Melon Gel 96-well Spin Plate according the manufacturer's instructions (Pierce). In short, 10µl of thawed sample was diluted in 90 µl of the equilibrated purification buffer on ice, then transferred onto Melon Gel support and incubated on a plate shaker at 500 rpm for 5 minutes. Centrifugation at 1,000 x g for 2 minutes was done to collect the purified IgG into the collection plate.

Protein concentrations of the collected IgG samples were measured by absorbance measures at 280nm using an Epoch Micro-Volume Spectrophotometer System (Biotec,USA). IgG-concentrations of all samples were concentration-adjusted and 0.6 mg/ml of samples were diluted 1:1 in PBS2x buffer with TritonX 0.2% and 6% skim milk powder for microarray analyses.

### Example 3: Microarray design

A protein-chip named "16k protein chip" from 15284 human cDNA expression clones derived from the Unipex cDNA expression library plus technical controls was generated. Using this 16k protein chip candidate markers were used to identify autoanti-body profiles suitable for unequivocal distinction of healthy, malign and benign colon tumors.

Protein-microarray generation and processing was using the Unipex cDNA expression library for recombinant protein expression in E.coli. His-tagged recombinant proteins were purified using Ni-metal chelate chromatography and proteins were spotted in duplicates for generation of the microarray using ARChipEpoxy slides.

### Example 4: Preparation, processing and analyses of protein microarrays

The microarray with printed duplicates of the protein marker candidates was blocked with DIG Easy Hyb (Roche) in a stirred glass tank for 30 minutes. Blocked slides were washed 3x for 5 minutes with fresh PBSTritonX 0.1% washing buffer with agitation. The slides were rinsed in distilled water for 15 seconds to complete the washing step and remove leftovers from the washing buffer. Arrays were spun dry at 900rpm for 2 minutes. Microarrays were processed using the Agilent Microarray Hybridisation Chambers (Agilent) and Agilent's gasket slides filled with 490 µl of the prepared sample mixture and processed in a hybridization oven for 4 h at RT with a rotation speed of 12. During this hybridization time the samples were kept under permanent rotating conditions to assure a homolog dispensation.

After the hybridization was done, the microarray slides were washed 3x with the PBSTritonX 0.1% washing buffer in the glass tank with agitation for 5 minutes and rinsed in distilled water for about 15 seconds. Then, slides were dried by centrifugation at 900 rpm for 2 minutes. IgG bound onto the features of the protein-microarrays were detected by incubation with cy5 conjugated Alexa Fluor® 647 Goat Anti-Human IgG (H+L) (Invitrogen, Lofer, Austria), diluted in 1:10,000 in PBSTritonX 0.1% and 3% skim milk powder using rotating conditions for 1h, with a final washing step as outlined above. Microarrays were then scanned and fluorescent data extracted from images (Fig. 1) using the GenePixPro 6.0 software (AXON).

### Example 5: Data analysis

Data were 1) quantil normalised and alternatively 2) normalised in DWD transformation for removal of batch effects, when samples were processed on microarrays in 4 different runs; data analyses was conducted using BRB array tools (web at linus.nci.nih.gov/BRB-ArrayTools.html) upon the 2 different normalization strategies (quantil and DWD normalized).

For identification of tumor marker profiles and classifer markers, class prediction analyses applying leave-one-out cross-validation was used. Classifiers were built for distinguishing each of the four classes of samples denoted "Carc" carcinoma patients, "HR" patients harboring high-risk polyps, "LR" patients harboring low-risk polyps, and "Contr" individuals with no carcinoma or polyps. In addition different combinations of classes were also built as listed in the table below (Tab. 1) and again class prediction analysis was conducted for differentiation of these different combinations.

**Table 1: Colon tumor marker Classifiers defined for separation of different indications (Carc.: Colon Carcinoma, Contr.: controls with no colon or polyp/tumor; LR/HR: low / high risk polyps; vs.... versus). The various examples upon data analyses after different normalization strategies A) and B) are given.**

| **Contrast analysed** | **A) examples with quantil normalisation** | **B) examples with DWD normalisation** |
|---|---|---|
| 1) Contr. vs. Carc & HR & LR | 7.1, 7.2 | 7.17, 7.18 |
| 2) Contr. vs. Carc. | 7.3-7.6 | 7.19-7.22 |
| 3) Contr. & LR vs. Carc. & HR | 7.7-7.9 | 7.23, 7.24 |
| 4) Carc. vs. Hr vs. LR vs. Contr. | 7.10, 7.11 | 7.25 |
| 5) Contr. vs. LR | 7.12 | 7.26 |
| 6) LR vs. HR | 7.13, 7.14 | 7.27 |
| 7) HR vs. Carc. | 7.15, 7.16 | 7.28, 7.29 |

### Example 6: Results Summary

For distinguishing 1) Contr vs "Carc-HR- LR", 2) Controls vs Carcinomas, or 3) Contr_LR vs Carc_HR, 47 genes were present in at least 5 classifier lists. The classification success with respect to different contrasts (differentiation of different patient classes and combinations thereof) and presence of 47 preferred List 2 markers is given in Table 3. As shown, the number of markers out of the 47 selected. It was also shown that using only isolated or only 2 markers from the present classifier sets enables correct classification of >60% (Example 8, Fig. 2 & 3). Therefore the marker-lists, subsets and single markers (antigens; proteins; peptides) are of particular diagnostic values.

In addition it has already been shown that peptides deduced from proteins or seroreactive antigens can be used for diagnostics and in the published setting even improve classification success (Syed 2012; Journal of Molecular Biochemistry; Vol 1, No 2, www.jmolbiochem.com/index.php/JmolBiochem/article/view/54).

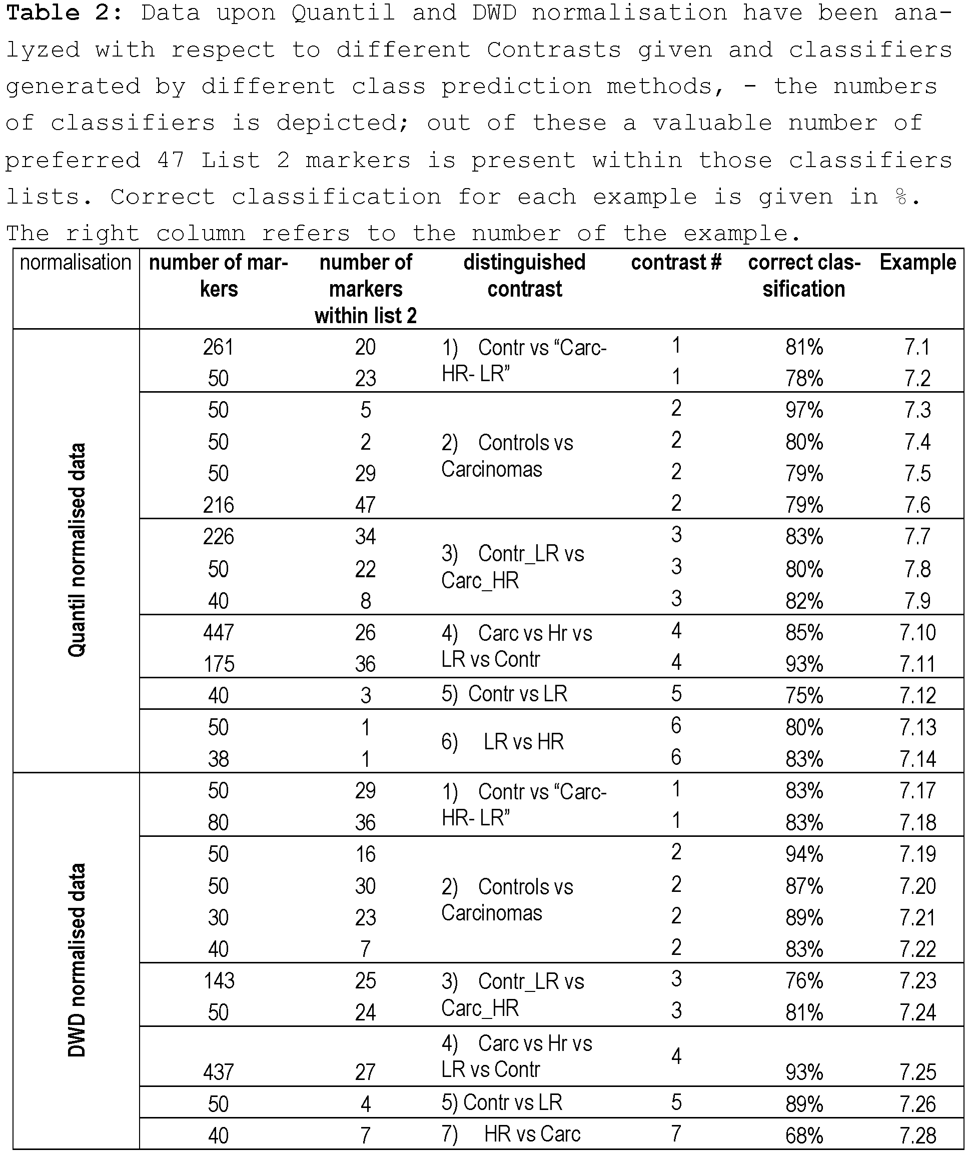

Different classifier lists have been elucidated for the "contrasts" listed in Table 1, - upon A) quantil normalization (QNORM) and B) DWD transformation.
Classifier markers (n=1150) were identified according to List 1. 441 markers were present in in both sets of A) and B) normalized data; 431 markers were present in classifier-sets upon A) quantil normalization, and 270 are present in classifiers upon DWD transformation.

Upon marker annotation 225 markers present an identical protein, 225 duplicates can be removed and remaining unique make up a list of 959 single Unigenes; thereof 374 are present in both sets of QNORM (A) and DWD (B) normalized data; 320 are present in classifiers upon QNORM, and an additional 265 are present in classifiers upon DWD transformation. Taken together based on the Unigene ID - 3 different markers are present in 17 classifiers, 2 markers in 16 classifiers, 7 markers in 14 classifiers etc.

### Example 7: Detailed Results

### Quantil-normalised data

### Example 7.1: Contr vs "Carc-HR-LR"- "grid of alpha levels" & 1.25 fold

The following markers were identified according to this example:
**List 3:** ACADVL, ACTL6B, ADAP1, ADCY1, ADD2, AK1, AKR1C4, ANGPTL2, ANXA6, APBB1, APBB1, ARHGEF1, ARL16, GDAP1L1, ASB13, ATP6V1H, ATXN10, BCS1L, BIN3, C10orf76, C16orf58, C16orf58, C17orf28, TMEM98, C17orf90, AFP, AFP, CALR, CALR, CAMK1D, CAPN2, CCDC64, CCDC88B, IK, CDH23, CDK16, CDK9, CHID1, CHID1, CKM, CLUAP1, COA5, COL6A3, CPNE9, CTAGE5, CUL1, CUX1, DBR1 (includes EG:323746), DDX19B, DDX41, DHX58, DNAH1, DOCK9, DOCK9, DPP3, DPYSL3, DRAP1, DRG2, EHMT2, EIF3E, EIF4A2, RPA1, EPS8L2, TACC2, EXOC6B, EXTL1, FAM108C1, FAM214A, HMGN2, FASN, FBXO44, FKBP15, FLII, FLNA, FLNA, FNTA, GBAS, GNPTG, GPCPD1, GPSM1, GRK6, GSS, HAUS4, HMGA1, HMGB2, HNRPDL, HSP90B1, HSPA8, SNAPIN, IDH3G, IDS, IFITM1, IFRD1, INA, IVNS1ABP, JARID2, KCNAB2, KCNG1, KIAA0368, KIAA1598, KPNA2, KPNB1, LAMB1, LGALS3, LMO4, LOC494127, HMGB2, LPCAT1, LPPR3, LRP5, LRRN2, LSM12 (includes EG:124801), LTBP3, MACF1, MED19, MICAL1, MRPL28 (includes EG:10573), MRPL38 (includes EG:303685), MTCH2, MYCBP2, NAPA (includes EG:108124), NARF, NELL2, NISCH, NMT2, NOA1, KPNA2, NRBP2, NOA1, NT5C, NUMA1, OAS3, OCRL, OGT, PAICS, PDDC1, PDIA4, PDZD4, PEA15, PEX5, PHF2, PHF8, PI4KA, PITPNC1, PKM2, PLAA, PKM2, PLAA, PLD2, PLXND1, POLN, PPIF, PPP1CA, PPP1R2, PPP4C, PSMC3, PSMD2, PSMD2, PTPN5, PTPN6, RAN, RIPK1, RNH1, RPL10A, RPL17, RPL24, RPL26, RPL26, RPN1, RRM1, RSL1D1, RUVBL2, SAT1, SCHIP1, SERPINB1, SFTPA1, SLC4A3, SKIV2L, SLU7 (includes EG:10569), SMURF2, SNRNP200, SNX1, SORL1, SPAG17, SPECC1L, SPP1 (includes EG:20750), STK25, TBCD, TBL3, TCERG1, TCF3, HSPA5, TFAP4, THAP7, VIM, TKT, TMCC2, TMEM12OA, TMSB10/TMSB4X, TPT1 (includes EG:100043703), TSC22D3, TUBA1B, TUBA1B, TUBB4A, TWF2, TYK2, UROD, PAICS, VAT1, VPS13C, VTI1B, WDR18, WDR35, YME1L1, YWHAQ, ZC3H10, ZNF12, ZNF354B, ZSWIM4.

Using microarray data for distinguishing "Contr" versus "Carcinoma & polyps" class prediction analysis was performed using feature selection upon "optimization of the grid of alpha levels" and "1.25 fold change of median intensities between classes". The 1-Nearest Neighbour Predictor gave the best correct classification of 81% using 216 markers.

Genes significantly different between the classes at the 0.01, 0.005, 0.001 and 0.0005 significance levels were used to build four predictors. The predictor with the lowest cross-validation mis-classification rate was selected. The best compound covariate classifier consisted of genes significantly different between the classes at the 5e-04 significance level. The best diagonal linear discriminant analysis classifier consisted of genes significantly different between the classes at the 5e-04 significance level. The best 1-nearest neighbor classifier consisted of genes significantly different between the classes at the 0.001 significance level. The best 3-nearest neighbors classifier consisted of genes significantly different between the classes at the 5e-04 significance level. The best nearest centroid classifier consisted of genes significantly different between the classes at the 5e-04 significance level. The best support vector machines classifier consisted of genes significantly different between the classes at the 0.01 significance level. The best Bayesian compound covariate classifier consisted of genes significantly different between the classes at the 5e-04 significance level. Only genes with the fold-difference between the two classes exceeding 1.25 were used for class prediction.

Repeated 1 times K-fold (K= 20) cross-validation method was used to compute mis-classification rate.

**Performance of classifiers during cross-validation.**

| | **Array id** | **Class label** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Compound Covariate Predictor Correct?** |
|---|---|---|---|---|---|---|---|---|---|
| **Mean percent of correct classification:** | | | **66** | **69** | **81** | **80** | **65** | **87** | **81** |

The following parameters can characterize performance of classifiers: Sensitivity, Specificity, Positive Predictive Value, Negative Predictive Value; Sensitivity is the probability for a class A sample to be correctly predicted as class A, Specificity is the probability for a non-class A sample to be correctly predicted as non-A, PPV is the probability that a sample predicted as class A actually belongs to class A, NPV is the probability that a sample predicted as non-class A actually does not belong to class A.

For each classification method and each class, these parameters are listed in the tables below

**Performance of the 1-Nearest Neighbor Classifier:**

| **Class** | **Sensitivity** | **Specificity** | **PPV** | **NPV** |
|---|---|---|---|---|
| **Care HR_LR** | 0.917 | 0.64 | 0.811 | 0.821 |
| **Control** | 0.64 | 0.917 | 0.821 | 0.811 |

**Performance of the 3-Nearest Neighbors Classifier:**

| **Class** | **Sensitivity** | **Specificity** | **PPV** | **NPV** |
|---|---|---|---|---|
| **Care HR_LR** | 0.964 | 0.52 | 0.771 | 0.897 |
| **Control** | 0.52 | 0.964 | 0.897 | 0.771 |

**Performance of the Support Vector Machine Classifier:**

| **Class** | **Sensitivity** | **Specificity** | **PPV** | **NPV** |
|---|---|---|---|---|
| **Carc_HR_LR** | 0.94 | 0.76 | 0.868 | 0.884 |
| **Control** | 0.76 | 0.94 | 0.884 | 0.868 |

### Example 7.2: "Carcinoma & polyps vs Contr" -25greedy pairs >1NN & SVM 78%

The following markers were identified according to this example:
**List 4:** ARHGAP21, ARHGEF1, ASB13, BCKDHA, BCS1L, C10orf76, C17orf28, TMEM98, C17orf90, AFP, AFP, CHD8, DEF8, EID1, RPA1, TACC2, ACTL6B, HMGN2, FASN, C10orf2, HAUS4, BTBD6, MRPL47, MTCH2, NARFL, NME2, NRBP2, NOA1, PDP1, PIK3R2, PSMA2, PSMC2, RAD1, RPL26, RPL37A, SBF2, SERPINA1, SLC4A3, MGAT4B, UBE2L3, UMPS, UROD, PAICS, VPS72 (includes EG:100001285), WDR13, ZNF668.

Alternatively to example 7.1 the "greedy pairs" strategy was used for class prediction, and it was possible to very efficiently build classifiers for distinguishing "Contr" versus "Carcinoma & polyps" (contrast 1). Using "25 greedy pairs" of features on arrays, the 1-Nearest Neighbor Predictor (1-NN) and the Support Vector Machines Predictor (SVM) enabled correct classification of 78% of samples.

Greedy pairs algorithm was used to select 25 pairs of genes. Repeated 1 times K-fold (K= 20) cross-validation method was used to compute mis-classification rate.

**Performance of classifiers during cross-validation.**

| | **Array id** | **Class label** | **Mean Number of genes in classifier** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Compound Covariate Predictor Correct?** |
|---|---|---|---|---|---|---|---|---|---|---|
| **Mean percent of correct classification:** | | | | **70** | **71** | **78** | **75** | **69** | **78** | **77** |

**Performance of the 1-Nearest Neighbor Classifier:**

| **Class** | **Sensitivity** | **Specificity** | **PPV** | **NPV** |
|---|---|---|---|---|
| **Care**_**HR_LR** | 0.929 | 0.52 | 0.765 | 0.812 |
| **Control** | 0.52 | 0.929 | 0.812 | 0.765 |

**Performance of the 3-Nearest Neighbors Classifier:**

| **Class** | **Sensitivity** | **Specificity** | **PPV** | **NPV** |
|---|---|---|---|---|
| **Carc_HR_LR** | 0.94 | 0.44 | 0.738 | 0.815 |
| **Control** | 0.44 | 0.94 | 0.815 | 0.738 |

### Example 7.3: "Carc vs. Contr" - 25greedy pairs >97% 1NN

The following markers were identified according to this example:
**List 5:** APBA3, C11orf80, CAMK1D, CCND1, CEP57, COL6A3, HSPA1A/HSPA1B, CSRNP1, CUL1, DDX19B, DENND5A, EHMT2, EIF3L, ERBB3 (includes EG:13867), TACC2, FBN1, HK1, IVNS1ABP, JMJD7-PLA2G4B, KIF3C, METTL2B, NAGLU, NCAN, NFKB2, NME2, PBX2, PDZD4, PLXNA1, PRKACA, PSMB6, RPL24, RPS7, RUVBL2, SGSH, SPRY1, TAGLN3, TNFAIP2, TSTA3, TTR, UMPS.

For contrast 2 the "greedy pairs" strategy was used for class prediction for the first 34 (17 carc; 17 contr) samples of run1, and it was possible to very efficiently build classifiers for distinguishing "Contr" versus "Carc". Using "25 greedy pairs" of features on arrays, the 1-Nearest Neighbor Predictor (1-NN) enabled best correct classification of 97% of samples.

Greedy pairs algorithm was used to select 25 pairs of genes. Leave-one-out cross-validation method was used to compute mis-classification rate.

**Performance of classifiers during cross-validation.**

| | **Array id** | **Class label** | **Mean Number of genes in classifier** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Compound Covariate Predictor Correct?** |
|---|---|---|---|---|---|---|---|---|---|---|
| **Mean percent of correct classification:** | | | | **91** | **91** | **97** | **97** | **97** | **91** | **91** |

**Performance of the 1-Nearest Neighbor Classifier:**

| **Class** | **Sensitivity** | **Specificity** | **PPV** | **NPV** |
|---|---|---|---|---|
| **Carcinoma** | 0.941 | 1 | 1 | 0.944 |
| **Control** | 1 | 0.941 | 0.944 | 1 |

**Performance of the 3-Nearest Neighbors Classifier:**

| **Class** | **Sensitivity** | **Specificity** | **PPV** | **NPV** |
|---|---|---|---|---|
| **Carcinoma** | 0.941 | 1 | 1 | 0.944 |
| **Control** | 1 | 0.941 | 0.944 | 1 |

**Performance of the Nearest Centroid Classifier:**

| **Class** | **Sensitivity** | **Specificity** | **PPV** | **NPV** |
|---|---|---|---|---|
| **Carcinoma** | 0.941 | 1 | 1 | 0.944 |
| **Control** | 1 | 0.941 | 0.944 | 1 |

### Example 7.4: "Carc vs. Contr" - 25greedy pairs - 80% 1NN

The following markers were identified according to this example:
**List 6:** A1BG, ACTR1B, ADCK3, AP3D1, ARAP1, CHST10, BCL6, TMEM98, C17orf90, AFP, AFP, C6orf136, COL8A2, CTNND2, DCAF15, GTF3A, ICAM3, IFNGR2, LMNA, LRRC4C, MAN2B1, MTA1, NPDC1, LOC440354, PMVK, POMGNT1, POTEE/POTEF, PPCDC, TSTA3, RBPJ, RPL7, SETD1B, SLMAP, SNTA1, STAB1, SUOX, TIAL1, TMCO7, TRIP12, TRPC4AP, ZCCHC14, ZNF589.

The "greedy pairs" strategy was used for class prediction of the first 34 (17 carc; 17 contr) samples processed in run2, and it was possible to very efficiently build classifiers for distinguishing "Contr" versus "Carc". Using "25 greedy pairs" of features on arrays, the 1-Nearest Neighbor Predictor (1-NN) enabled best correct classification of 80% of samples.

Greedy pairs algorithm was used to select 25 pairs of genes. Leave-one-out cross-validation method was used to compute mis-classification rate.

**Performance of classifiers during cross-validation.**

| | **Array id** | **Class label** | **Mean Number of genes in classifier** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Compound Covariate Predictor Correct?** |
|---|---|---|---|---|---|---|---|---|---|---|
| **Mean percent of correct classification:** | | | | **77** | **70** | **73** | **80** | **73** | **73** | **80** |

**Performance of the 1-Nearest Neighbor Classifier:**

| **Class** | **Sensitivity** | **Specificity** | **PPV** | **NPV** |
|---|---|---|---|---|
| **Carcinoma** | 0.867 | 0.6 | 0.684 | 0.818 |
| **Control** | 0.6 | 0.867 | 0.818 | 0.684 |

### Example 7.5: Carc vs Contr -25 greedy pairs - 1NN 79 %

The following markers were identified according to this example:
**List 7:** ACTL6B, ARHGEF10L, ATP5H, ATXN2, TMEM98, C17orf90, AFP, AFP, C18orf32, CTDP1, DEF8, DPYSL5, EIF4A2, TACC2, ACTL6B, PLEKHO1, EXOSC8, HAUS4, BTBD6, LRRC4C, MACF1, MAN2B1, MTCH2, MTSS1L, NARFL, NBEAL2, NHEJ1, OTUD5, PDP1, PKM2, PLAA, PLEKHA5, POLN, PRKCSH, RAD1, RPL37A, RSL1D1, RSL1D1, SAFB2, SERPINA1, SPRY1, TPX2, UROD, UROD, PAICS, VPS18.

The "greedy pairs" strategy was used for class prediction of all carc & contr samples, and it was possible to very efficiently build classifiers for distinguishing "Contr" versus "Carc". Using "25 greedy pairs" of features on arrays, the 1-Nearest Neighbor Predictor (1-NN) enabled best correct classification of 79% of samples.

Greedy pairs algorithm was used to select 25 pairs of genes. Repeated 1 times K-fold (K= 20) cross-validation method was used to compute mis-classification rate.

**Performance of classifiers during cross-validation.**

| | **Array id** | **Class label** | **Mean Number of genes in classifier** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Compound Covariate Predictor Correct?** |
|---|---|---|---|---|---|---|---|---|---|---|
| **Mean percent of correct classification:** | | | | **77** | **72** | **79** | **72** | **78** | **73** | **81** |

**Performance of the 1-Nearest Neighbor Classifier:**

| | | | | |
|---|---|---|---|---|
| **Class** | **Sensitivity** | **Specificity** | **PPV** | **NPV** |

| | | | | |
|---|---|---|---|---|
| **Carcinoma** | (0.898 | 0.68 | 0.733 | 0.872 |
| **Control** | 0.68 | 0.898 | 0.872 | 0.733 |

**Performance of the 3-Nearest Neighbors Classifier:**

| **Class** | **Sensitivity** | **Specificity** | **PPV** | **NPV** |
|---|---|---|---|---|
| **Carcinoma** | 0.898 | 0.54 | 0.657 | 0.844 |
| **Control** | 0.54 | 0.898 | 0.844 | 0.657 |

### Example 7.6: "Carc vs Contr" -grid of alpha; 1.25 fold > SVM79%

The following markers were identified according to this example:
**List 8:** ABCA3, ACAA2, ACTL6B, ACTL6B, ADNP, AHCY, ALB, AMBRA1, APLP1, ARAP1, CHST10, ARAP2, ARHGAP21, ZNF259, ARHGAP44, ARHGEF1, ARHGEF10L, ARHGEF17, ASCL1, ATP5H, ATXN2, AXIN1, BCAS2, BCS1L, BGN, C10orf76, C17orf28, TMEM98, C17orf90, AFP, AFP, C18orf32, C20orf20, PECAM1, C5orf25, C6orf136, CCDC64, CCT8, CHD8, CLCN6, CLTA, COL3A1, CPNE6, CTDP1, CUL1, CYC1, DBI, DCTPP1, DDX41, DEF8, DOCK9, DOCK9, DPP3, DPYSL5, DYNC1H1, EEF1A1, EFTUD1, EHMT2, EHMT2, EID1, RPA1, EPC1, EPS8, TACC2, ETS1, ACTL6B, PLEKHO1, EXOSC8, FAM149A, FAM204A, FHOD1, FLII, FLNB, GNAO1, GNPTG, GP1BB, GPCPD1, GTDC1, C10orf2, HAUS4, HERC4, HK1, HMGA1, JMJD4, HNRNPK, BTBD6, HSPA9, HTRA1, IDS, IMP4, ITSN1, KAT8, KCNIP1, LCP1, LDOC1, LMF2, LMNA, HMGB2, LRRC4C, LSM12 (includes EG:124801), LTBP3, MACF1, MAPK10, MAPRE1, MAST2, MRPL47, MTCH1, MTCH2, MTSS1L, NARFL, NASP, NBEAL2, NHEJ1, NKTR, NME2, NRBP2, NOA1, NSMCE2, NUMA1, PAPLN, PATZ1, PDIA3, PDP1, PFKL, PI4KA, PIK3CD, PIK3R2, PKM2, PLAA, PKM2, PLAA, PLCG1, PLEKHA5, PLXNA1, PLXNB1, PLXND1, POMT1, PPP1R2, PRKCSH, PSMB1, PTPRO, QTRT1, RBM4, RBM4, RHOB, RNF40, RPL13, RPL26, RPL27, RPL37A, RPS10, MICAL1, RSL1D1, RSL1D1, KLHL23/PHOSPHO2-KLHL23, RTN4 (includes EG:57142), RUVBL2, SAFB2, SCARF2, SDCBP, SERPINA1, SERPINH1, SGSH, SLC4A3, DARS, SLC4A2, SLC4A3, SNRNP40, SPAG1, SPAG17, SPRY1, SPTLC1, STAU1, STK17A, MGAT4B, TIAM2, TMCC2, TMEM123 (includes EG:114908), TP53 (includes EG:22059), TP53 (includes EG:22059), TP53BP1, TPX2, TRIOBP, TSTA3, TTYH3, TUBGCP2, UBE2L3, UROD, UROD, PAICS, VPS18, WDR73, YARS, YARS, YPEL1, YY1, ZNF12, ZNF358, ZNF512B, ZNF668.

Using feature selection upon "optimization of the grid of alpha levels" and "1.25 fold change of median intensities between classes" 7 classifier lists using different numbers of markers by the specific classification model (at an optimized significance level) were identified.

Genes significantly different between the classes at the 0.01, 0.005, 0.001 and 0.0005 significance levels were used to build four predictors. The predictor with the lowest cross-validation mis-classification rate was selected. The best compound covariate classifier consisted of genes significantly different between the classes at the 0.001 significance level. The best diagonal linear discriminant analysis classifier consisted of genes significantly different between the classes at the 0.001 significance level. The best 1-nearest neighbor classifier consisted of genes significantly different between the classes at the 0.005 significance level. The best 3-nearest neighbors classifier consisted of genes significantly different between the classes at the 0.001 significance level. The best nearest centroid classifier consisted of genes significantly different between the classes at the 0.005 significance level. The best support vector machines classifier consisted of genes significantly different between the classes at the 0.005 significance level. The best Bayesian compound covariate classifier consisted of genes significantly different between the classes at the 5e-04 significance level.

Only genes with the fold-difference between the two classes exceeding 1.25 were used for class prediction. Repeated 1 times K-fold (K= 20) cross-validation method was used to compute mis-classification rate.

**Performance of classifiers during cross-validation.**

| | **Array id** | **Class label** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Compound Covariate Predictor Correct?** |
|---|---|---|---|---|---|---|---|---|---|
| **Mean percent of correct classification:** | | | **75** | **74** | **74** | **75** | **73** | **79** | **77** |

**Performance of the 1-Nearest Neighbor Classifier:**

| **Class** | **Sensitivity** | **Specificity** | **PPV** | **NPV** |
|---|---|---|---|---|
| **Carcinoma** | 0.918 | 0.56 | 0.672 | 0.875 |
| **Control** | 0.56 | 0.918 | 0.875 | 0.672 |

**Performance of the 3-Nearest Neighbors Classifier:**

| **Class** | **Sensitivity** | **Specificity** | **PPV** | **NPV** |
|---|---|---|---|---|
| **Carcinoma** | 0.939 | 0.56 | 0.676 | 0.903 |
| **Control** | 0.56 | 0.939 | 0.903 | 0.676 |

### Example 7.7: "Carc_HR vs Contr_LR" p<0.005 > SVM 83%

The following markers were identified according to this example:
**List 9:** AAAS, ABCE1, ACAA2, ACAP1, ACIN1, ACTL6B, ADNP, ALB, APBB1IP, ARHGAP21, ARHGAP25, ARHGEF10L, ARHGEF17, ARID5A, ASAP1, ASB13, ATP5D, AXIN1, AZI2, BAG6, BCAS2, BCKDHA, BCLAF1, C10orf76, C17orf28, TMEM98, C17orf90, AFP, AFP, C18orf32, PECAM1, CDH10, CDK16, CLDN5, CLTA, CLTA, CLUAP1, CNPY3, CRABP1, CSF1R, CTDP1, CUL1, CYHR1, DCAF13, DDX27, DDX41, DEF8, DHX36, DLG5, DLX2, EDC3 (includes EG:315708), EEF1A1, EID1, EIF4A2, TACC2, EVI5L, ACTL6B, PLEKHO1, EXOSC4, EXOSC8, EXOSC8, FAM108A1, FAM149A, FAM192A, FAM209B, FAM59B, FBRS, FHOD1, GALE, GNAO1, GPCPD1, GPN1, GTF2B, HARS, HAUS4, HBP1, HERPUD1, HMG20B, HMGN2, JMJD4, HNRNPA0, HNRNPK, HNRNPR, HNRPLL, ELAC2, BTBD6, HTRA1, INA, INPP5K, KIAA0753, KPNB1, LAMA5, LAMB1, LARP1, LMTK2, LONP1, LRRC4C, MAGED2, MAST2, MCM6, MCM9, MGAT4B, MIIP, MYL6, MYO1D, MYO1D, MYO5A, NARFL, NCOR1, NDUFA13, NME2, NRBP2, NOA1, NUMA1, OTUD5, PAICS, PDIA3, LOC440354, PIK3CD, PJA1, PKM2, PLAA, PKP3, PLXNB1, PLXND1, POLE2, POLRMT, POMT1, PPP5C, PQBP1, PRDX1, PRKCSH, PSKH1, PSMA2, PSMB5, PSMD10, PTPRF, RAB3IL1, RBM4, RBM4, RBM4, RBM4, REC8 (includes EG:290227), RHBDD2, RHOB, RNF220, RNF40, RP9, RPL13, RPL17, RPL22, RPL37A, SAFB2, SERPINA1, SERPINH1, SETD2, SFTPB, DARS, SH3BP2, SLC25A20, SPOCK2, SPRY1, SPTAN1, SPTLC1, STMN4, STT3B, TBC1D9B, THOC3, TIAM2, TMCC2, TMEM57, TNFRSF6B, TP53BP1, TPM3, ZWINT, TTC5, TUBB, TUBB3, TUBGCP2, TWF1 (includes EG:19230), UBC, UBE2L3, UBE2N, ULK1, UROD, UROD, VARS, PAICS, VPS18, WASH1/WASH5P, WDR13, WDR73, WDR77, XAB2, YARS, YPEL1, YY1, ZFYVE1, ZNF133, ZNF410, ZNF668, ZNF695, ZXDC.

Using feature selection upon "Genes significantly different between the classes at 0.005 significance level" and " 1.25 fold change of median intensities between classes" 7 classifier lists using different numbers of markers by the specific classification model were identified. The Support Vector Machines Predictor gave the best correct classification of 83% using 226 markers.

Genes significantly different between the classes at 0.005 significance level were used for class prediction. Repeated 1 times K-fold (K= 20) cross-validation method was used to compute mis-classification rate.

**Performance of classifiers during cross-validation.**

| | **Array id** | **Class label** | **Mean Number of genes in classifier** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Compound Covariate Predictor Correct?** |
|---|---|---|---|---|---|---|---|---|---|---|
| **Mean percent of correct classification:** | | | | **69** | **67** | **73** | **69** | **69** | **83** | **78** |

**Performance of the Support Vector Machine Classifier:**

| **Class** | **Sensitivity** | **Specificity** | **PPV** | **NPV** |
|---|---|---|---|---|
| **Carc_HR** | 0.851 | 0.806 | 0.814 | 0.844 |
| **Cont_LR** | 0.806 | 0.851 | 0.844 | 0.814 |

### Example 7.8: "Carc_HR vs Contr_LR" 25greedy pairs >SVM 80%

The following markers were identified according to this example:
**List 10:** ABCE1, ACAA2, APBB1IP, ARHGAP21, ARHGEF10L, BCKDHA, C10orf76, C18orf32, CDK16, COMMD9, DHX36, DLG5, EID1, ACTL6B, PLEKHO1, GTF2B, HAUS4, JMJD4, HNRNPR, INPP5K, LAMB1, LRRC4C, NARFL, PDIA3, PIK3CD, PJA1, PKM2, PLAA, PLXND1, PPP5C, PSKH1, RNF40, RPL22, RPL37A, SERPINA1, SPRY1, TBC1D9B, TIAM2, TMCC2, VARS, PAICS, VPS18, YPEL1, ZNF668.

The "greedy pairs" strategy was used for class prediction of all Carc_HR vs Contr_LR samples, and it was possible to very efficiently build classifiers for distinguishing classes. Using "25 greedy pairs" of features on arrays, the Support Vector Machines Predictor (SVM) enabled best correct classification of 80% of samples.

Greedy pairs algorithm was used to select 25 pairs of genes. Repeated 1 times K-fold (K= 20) cross-validation method was used to compute mis-classification rate.

**Performance of classifiers during cross-validation.**

| | **Array id** | **Class label** | **Mean Number of genes in classifier** | **Compound Covariate Predictor Correct?** | **Diagonal Linear Discriminant Analysis Correct?** | **1-Nearest Neighbor** | **3-Nearest Neighbors Correct?** | **Nearest troid Centroid Correct? rect?** | **Support Vector Machines Correct?** | **Bayesian Compound Covariate Predictor Correct?** |
|---|---|---|---|---|---|---|---|---|---|---|
| **Mean percent** | | | | **70** | **72** | **73** | **75** | **71** | **80** | **79** |
| **of correct classification:** | | | | | | | | | | |

**Performance of the Support Vector Machine Classifier:**

| **Class** | **Sensitivity** | **Specificity** | **PPV** | **NPV** |
|---|---|---|---|---|
| **Carc_HR** | 0.821 | 0.776 | 0.786 | 0.812 |
| **Cont_LR** | 0.776 | 0.821 | 0.812 | 0.786 |

### Example 7.9: "Carc_HR vs Contr_LR" 40Recursive features >SVM 82%

The following markers were identified according to this example:
**List 11:** ACAA2, APBB1IP, ASAP1, PECAM1, IK, CDT1, CHCHD8, CPNE2, DTNBP1, TACC2, ACTL6B, GNAO1, GRK6, HDAC6, ELAC2, LRIG1, LRRC16B, LRRC4C, MYO1C, NME2, KPNA2, PDIA3, LOC440354, PRRT2, PSKH1, RPL24, SNTA1, STMN4, TIAL1, TMCC2, TRAPPC3, GPX4, UBC, YLPM1, ZCCHC11, ZNF232.

Alternatively to the the "greedy pairs" strategy for class prediction of all Carc_HR vs Contr_LR samples, the "Recursive feature" extraction strategy was used and selection of 40 features enabled 82% correct classification using the SVM method.

Recursive Feature Elimination method was used to select 40 genes. Repeated 1 times K-fold (K= 20) cross-validation method was used to compute mis-classification rate.

**Performance of classifiers during cross-validation.**

| | **Array id** | **Class label** | **Mean Number of genes in classifier** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Compound Covariate Predictor Correct?** |
|---|---|---|---|---|---|---|---|---|---|---|
| **Mean percent of correct classification:** | | | | **78** | **74** | **79** | **74** | **73** | **82** | **79** |

**Performance of the Support Vector Machine Classifier:**

| **Class** | **Sensitivity** | **Specificity** | **PPV** | **NPV** |
|---|---|---|---|---|
| **Carc_HR** | 0.806 | 0.836 | 0.831 | 0.812 |
| **Cont_ LR** | 0.836 | 0.806 | 0.812 | 0.831 |

### Example 7.10: "Carc, HR, LR, Contr" - SVM >85%

The following markers were identified according to this example:
**List 12, part a):** ABCE1, ACAA2, AFMID, AKR1C4, AKR1C4, AKT2, AP2A1, BTBD7, APRT, ARHGEF10L, BMS1P5, C17orf90, AFP, CHD3, CLU, COPS6, CTC1, DCAF11, DLG5, ELP2, EPC1, FIBP, FN3K, GBP2 (includes EG:14469), GMPPB, HIPK3, IL6ST, INTS1, ITGAL, LRIG1, MTA2, MVD, MYO5A, NKRF, NOMO1 (includes others), OTUB1, PDE4D, PERI, PHF3, PLXNA3, POR, PSKH1, RABEPK, RNF40, RPL22, SBF1, SEMA3F, SEPT1, SERPINB9, SGSH, SPRN, SPSB3, TAGLN3, TBC1D9B, TBCB, TP53 (includes EG:22059), TSC2, TUBB6, USP39, VARS, WDR63, ZNF277, ZNF672.
**List 12, part b):** ABCA2, ABCE1, ACAA2, ACAP1, ACD, ACSL3, ACTB, ADCY1, ADRBK1, AGPAT6, AHSG, AKR1B1, AKT1, ALB, ANAPC5, IGSF9, ANXA6, AP2A1, BTBD7, APBA3, APRT, ARF4, ARF5, ARHGAP25, ARHGEF2, ARMCX1, ASB13, ATP5D, ATP8B3, BCLAF1, BCS1L, BMS1P5, BSDC1, BTBD11, C18orf21, C18orf32, PECAM1, C8orf33, C8orf33, CALR, CAPN2, CAPN2, NDUFB7, CCDC64, CCDC88B, CCL28, CCT8, CDC37, CDK16, CDK5RAP3, CHID1, CNPY3, COMMD9, COX7A2L, CPNE1, CPNE5, CRABP1, CSF1R, CSNK2B, CTBP2, CTBP2, DCAF10, DCTN3, DCTPP1, DDIT4, DDX27, DDX41, DDX56, DEF8, DHX8, DHX8, DLG5, DLX2, DPP3, DRAP1, DSE, DVL1, EEF1A1, EEF1A1, EEF1A1, EIF2B5, RPA1, ELMO1, ELP3, EPN1, EPN1, TACC2, ERP44, ESYT1, ACTL6B, MEAF6, FAM209B, FAM59B, KIAA1109, FAM65A, FCHSD1, FLII, FNTA, FXYD6, G3BP2, GAL3ST4, GGA1 (includes EG:106039), GHITM, GIT1 (includes EG:216963), GOLGA4, GSK3A, GTF3C5, HLA-A, HMGA1, HMGCL, HMGN2, JMJD4, HNRNPH1, HSD17B4, HSP90B1, HSPA1A/HSPA1B, IFRD1, INPP5K, JAG1, KCTD13, KHSRP, KIAA1244, KIF19, LAMP1, LCP1, LMTK2, LOC285463, LPCAT1, LSM14A, LTBP3, MAF1 (includes EG:315093), MAP1A, TCF19, MAPK8IP1, MAPK8IP3, MAST2, MEAF6, MGAT4B, MGAT4B, MIB2, MIIP, MLL4, MPI, MRPS11, MTCH1, MTCH2, RPL17, MUC5AC/MUC5B, NARF, NARFL, NBEAL2, NCF1, NCS1, NDUFA13, NDUFB10, NDUFS2, NIPSNAP1, NME2, NME2, NOMO1 (includes others), NPDC1, NR4A1, NT5C, NTAN1, NUMA1, ODC1, OLFML2A, ALB, PAICS, PCDH9, PDIA3, PI4KA, PIK3CD, PJA1, PKM2, PLAA, PKM2, PLAA, PLCE1, PLXNA3, PLXND1, PPID, PPM1F, PPP1R15A, PPP1R18, PRDX1, PRDX1, PRKCSH, PRKCZ, PRR3, PRRT1, PSAT1, PSKH1, PSMA2, PSMA2, PSME1, PSME1, ARHGDIA, PTPN12, PTPN23, PTPRS, BAD, PTPRS, BAD, HAPLN3, RANBP1, RBM15, RBM26, RBM4, RBM4, RC3H2, RHBDD2, RNF220, RPL10, RPL13, RPL22, RPL22, RPL26, RPL28, RPL37A, RPL7, RRP9, RTKN, RUSC1, SBF2, SCAF8, SCO1, SCOC, SDCBP, GRK6, SERBP1, SERPINA1, SETD2, SEZ6L2, SLC4A3, DARS, SLA, VIM, SMCHD1, SNIP1, SRA1, SRSF1, SRSF4, ST6GALNAC1, STOM, STX6, SUV420H1, SYS1 (includes EG:336339), TADA2B, MGAT4B, TLE1, TLE3, PXN, TMEM57, TMSB10/TMSB4X, TNFRSF25, TOE1, TOPORS, TPM3, ZWINT, TPM3, ZWINT, TRIM27, TTC3, TUBA1B, TUBA4A, TUBB, TUBGCP2, TWF2, UBB, UBE2N, UBXN1, UROD, TUBA1B, USP33, PAICS, VPS13C, VPS26A, WDR13, WDR35, WDR6, XYLT1, YPEL1, YY1, ZEB1, ZNF133, ZNF174, ZNF238, ZNF300, ZNF423, ZNF605, ZNF668.
**List 12, part c):** C10orf118, G6PD, GMIP, SNAPIN, HSPBAP1, RSL1D1, TCEA2, TPM3, ZWINT, TSPAN7.

Exemplifying the separation of all 4 different sample-classes, a "Binary tree" prediction analysis was conducted. As illustrated in that example the 1st classification node separates "Low Risk" from all the remaining classes using the markers of list 12, part a) at a "Mis-classification rate" (MCR) of 15.7%, then "controls" are distinguished from "Carcinoma & High Risk" by the markers of list 12, part b) (MCR=13.5%) and in the 3rd node "Carcinoma" are distinguished from "High Risk" by the markers of list 12, part c) (MCR=14.7%).

The markers of List 12, part a) can be used independently from the markers of list 12 parts b) and c) to distinguish LR from the combined group of cancer, healthy controls and HR polyps. Markers of list 12 parts b) and c) can be used to distinguish the indicated groups of classes (medical indications) but work best if the groups have been preselected by using the distinguishing markers of list 12a) in a previous step to remove the LR polyps from the question to be solved.

Binary Tree Classification algorithm was used. Feature selection was based on the univariate significance level (alpha = 0.001). The support vector machine classifier was used for class prediction. There were 3 nodes in the classfication tree. 10-fold cross-validation was performed.

Cross-validation error rates for a fixed tree structure shown below.

| **Node** | **Group 1 Classes** | **Group 2 Classes** | **Mis-classification rate (%)** |
|---|---|---|---|
| **1** | Carcinoma, Control, High Risk | Low Risk | 15.7 |
| **2** | Carcinoma, High Risk | Control | 13.5 |
| **3** | Carcinoma | High Risk | 14.7 |

Then the following parameters can characterize performance of classifiers: Sensitivity, Specificity, Positive Predictive Value (PPV), Negative Predictive Value (NPV)

These parameters are listed in the table below

| **Class** | **Sensitivity** | **Specificity** | **PPV** | **NPV** |
|---|---|---|---|---|
| **Carcinoma** | 0.706 | 0.849 | 0.6 | 0.9 |
| **Control** | 0.556 | 0.923 | 0.714 | 0.857 |
| **High Risk** | 0.647 | 0.868 | 0.611 | 0.885 |
| **Low Risk** | 0.5 | 0.846 | 0.529 | 0.83 |

### Example 7.11: "Carc, HR, LR, Contr" - SVM++

The following markers were identified according to this example:
**List 13, part a):** ABCA3, ADNP, AHCY, ALB, AMBRA1, ARHGAP21, ASCL1, ATP5H, AXIN1, BCS1L, BGN, C10orf76, C17orf28, TMEM98, C17orf90, AFP, AFP, C18orf32, PECAM1, C6orf136, CCDC64, CHD8, CLTA, COL3A1, CPNE6, CYC1, DCTPP1, DDX41, DEF8, DOCK9, DOCK9, DPP3, EEF1A1, EHMT2, EID1, RPA1, EPC1, EPS8, TACC2, ETS1, ACTL6B, EXOSC8, FLII, GPCPD1, C10orf2, HERC4, HMGA1, JMJD4, BTBD6, IDS, IMP4, ITSN1, KCNIP1, LCP1, LDOC1, HMGB2, LRRC4C, LSM12 (includes EG:124801), LTBP3, MACF1, MAST2, MRPL47, MTCH2, MTSS1L, NARFL, NASP, NBEAL2, NKTR, NME2, NRBP2, NOA1, NSMCE2, NUMA1, PATZ1, PDIA3, PDP1, PI4KA, PIK3CD, PIK3R2, PKM2, PLAA, PKM2, PLAA, PLCG1, PLXNB1, PLXND1, POMT1, PPP1R2, PRKCSH, PSMB1, RBM4, RBM4, RPL13, RPL26, RPL37A, SAFB2, SCARF2, SDCBP, SERPINA1, SLC4A3, DARS, SPRY1, SPTLC1, MGAT4B, TRIOBP, TUBGCP2, UBE2L3, UROD, UROD, PAICS, YARS, YARS, YPEL1, YY1, ZNF12, ZNF668.
**List 13, part b):** ACAA2, ARHGEF10L, C18orf32, CLTA, DBI, DCTPP1, EXOSC8, FLII, GNAO1, C10orf2, HNRNPK, LMNA, LRRC4C, LTBP3, MAST2, NARFL, NKTR, NUMA1, PIK3CD, PKM2, PLAA, PLXND1, RNF40, RPL26, RPL37A, RUVBL2, SGSH, TIAM2, TMCC2, TP53 (includes EG:22059), WDR73, YPEL1, YY1, ZNF512B.
**List 13, part c):** EFTUD1, ETS1, LRRC4C, NME2, PI4KA, PLEKHA5, QTRT1, RSL1D1, RSL1D1, TMEM183A.

For removal of any bias which might have been introduced upon the experimental design, the "Binary tree" prediction approach was repeated only to the experimental runs when all the 4 classes of samples were represented in two experiments. As expected classification success did improve; following numbers were obtained: As illustrated in the example the 1st classification node separates "Controls" from all the remaining classes using the markers of list 13, part a) at a "Mis-classification rate" (MCR) of 7.1%, then "Low Risk polyps" are distinguished from "Carcinoma & High Risk" by the markers of list 13, part b) (MCR=17.3%) and in the 3rd node "Carcinoma" are distinguished from "High Risk" by the markers of list 13, part c) (MCR=8.8%).

The markers of List 13, part a) can be used independently from the markers of list 13 parts b) and c) to distinguish health controls from the combined group of cancer, LR polyps and HR polyps. Markers of list 13 parts b) and c) can be used to distinguish the indicated groups of classes (medical indications) but work best if the groups have been preselected by using the distinguishing markers of list 13a) in a previous step to remove the healthy controls from the question to be solved.

Binary Tree Classification algorithm was used. Feature selection was based on the univariate significance level (alpha = 0.05) The support vector machine classifier was used for class prediction. There were 3 nodes in the classfication tree. 10-fold cross-validation was performed.

Cross-validation error rates for a fixed tree structure are shown below

| **Node** | **Group 1 Classes** | **Group 2 Classes** | **Mis-classification rate (%)** |
|---|---|---|---|
| **1** | Carcinoma High Risk, Low Risk | Control | 7.1 |
| **2** | Carcinoma, High Risk | Low Risk | 17.3 |
| **3** | Carcinoma | High Risk | 8.8 |

| **Class** | **Sensitivity** | **Specificity** | **PPV** | **NPV** |
|---|---|---|---|---|
| **Carcinoma** | 0.647 | 0.774 | 0.478 | 0.872 |
| **Control** | 0.611 | 0.981 | 0.917 | 0.879 |
| **High Risk** | 0.647 | 0.849 | 0.579 | 0.882 |
| **Low Risk** | 0.444 | 0.865 | 0.533 | 0.818 |

### Example 7.12: "Contr vs LR" - 40 recursive feature extr >75%

The following markers were identified according to this example:
**List 14:** AP1B1, ARPP21, ATP2A3, BMS1P5, CCL28, CDK16, DCTPP1, DDX19A, ELAVL1, TACC2, ITGAL, LRPAP1, MAGED1, MAST2, MYO5A, NAV2, NEFL, PDP1, PEX19, PFKL, PKM2, PLAA, PLEKHM2, MED4 (includes EG:29079), PNCK, PPP2R5C, RNF10, ACO2 (includes EG:11429), SEPT1, TBC1D9B, TRMT2A, WDR5, ZNF277, ZNF514.

For example the "recursive feature" strategy was used for class prediction of all Contr vs LR samples, and it was possible to very efficiently build a classifier for distinguishing classes. Using 40 recursive features on arrays, all the predictors enabled correct classification of 75% of samples.

Recursive Feature Elimination method was used to select 40 genes. Repeated 1 times K-fold (K= 20) cross-validation method was used to compute mis-classification rate.

**Performance of classifiers during cross-validation.**

| | **Array id** | **Class label** | **Mean Number of genes in classifier** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Compound Covariate Predictor Correct?** |
|---|---|---|---|---|---|---|---|---|---|---|
| **Mean percent of correct classification:** | | | | **75** | **75** | **75** | **75** | **75** | **75** | **75** |

| **Class** | **Sensitivity** | **Specificity** | **PPV** | **NPV** |
|---|---|---|---|---|
| **Control** | 0.833 | 0.667 | 0.714 | 0.8 |
| **Low Risk** | 0.667 | 0.833 | 0.8 | 0.714 |

### Example 7.13: LR vs HR 25 greedy pairs > SVM=80%

The following markers were identified according to this example:
**List 15:** ABCE1, AKAP17A, AP2A1, BTBD7 BMS1P5, CELSR1, CENPT, CHD3, CYB5R3, DLG5, FCHSD1, GTF2B, HIST1H2AC, HNRNPM, HNRNPR, ITGA5, JAG1, KIF21A, MAP4, NBPF11 (includes others), NDRG1, NDUFA13, NDUFS5, PHF3, PREP, RALGDS, REEP2, RNF213, RNF40, RPL17, RPL22, RUFY1, SEPT1, SETD2, SMCHD1, SORD, NAP1L1, SYS1 (includes EG:336339), TPM3, ZWINT, UBXN7, WDR62, ZNF672. The "greed pairs" strategy was used for class prediction of all LR vs HR samples, and a classifier for distinguishing classes was defined. Using 50 features on arrays, the nearest neighbor (NN) and SVM predictors enabled correct classification of 80% of samples.

Greedy pairs algorithm was used to select 25 pairs of genes. Repeated 1 times K-fold (K= 20) cross-validation method was used to compute mis-classification rate.

**Performance of classifiers during cross-validation.**

| | **Array id** | **Class label** | **Mean Number of genes in classifier** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Compound Covariate Predictor Correct?** |
|---|---|---|---|---|---|---|---|---|---|---|
| **Mean percent of correct classification:** | | | | **77** | **71** | **80** | **80** | **77** | **80** | **76** |

**Performance of the 1-Nearest Neighbor Classifier:**

| **Class** | **Sensitivity** | **Specificity** | **PPV** | **NPV** |
|---|---|---|---|---|
| **High Risk** | 0.824 | 0.778 | 0.778 | 0.824 |
| **Low Risk** | 0.778 | 0.824 | 0.824 | 0.778 |

**Performance of the 3-Nearest Neighbors Classifier:**

| **Class** | **Sensitivity** | **Specificity** | **PPV** | **NPV** |
|---|---|---|---|---|
| **High Risk** | 0.824 | 0.778 | 0.778 | 0.824 |
| **Low Risk** | 0.778 | 0.824 | 0.824 | 0.778 |

**Performance of the Support Vector Machine Classifier:**

| **Class** | **Sensitivity** | **Specificity** | **PPV** | **NPV** |
|---|---|---|---|---|
| **High Risk** | 0.882 | 0.722 | 0.75 | 0.867 |
| **Low Risk** | 0.722 | 0.882 | 0.867 | 0.75 |

### Example 7.14: LR vs HR 19 greedy pairs > CCP, 1NN=83%

The following markers were identified according to this example:
**List 16:** ABCE1, AP2A1, BTBD7, CELSR1, CENPT, CYB5R3, DLG5, FCHSD1, GTF2B, HIST1H2AC, HNRNPM, HNRNPR, KIF21A, MAP4, NBPF11 (includes others), NDRG1, PHF3, PREP, REEP2, RNF213, RNF40, RPL17, RPL22, RUFY1, SMCHD1, SORD, NAP1L1, SYS1 (includes EG:336339), TPM3, ZWINT, UBXN7, WDR62, ZNF672.

Then the "recursive feature" strategy for class prediction of all Carc vs HR samples was used, and it was possible to very efficiently build a classifier for distinguishing classes. Using 40 recursive features on arrays, the CCVP, SVM and BCCVP predictor enabled correct classification of 85% of samples.

Greedy pairs algorithm was used to select 19 pairs of genes. Repeated 1 times K-fold (K= 20) cross-validation method was used to compute mis-classification rate.

**Performance of classifiers during cross-validation.**

| | **Array id** | **Class label** | **Mean Number of genes in classifier** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Compound Covariate Predictor Correct?** |
|---|---|---|---|---|---|---|---|---|---|---|
| **Mean percent of correct classification:** | | | | **74** | **71** | **83** | **80** | **77** | **77** | **76** |

**Performance of the 1-Nearest Neighbor Classifier:**

| **Class** | **Sensitivity** | **Specificity** | **PPV** | **NPV** |
|---|---|---|---|---|
| **High Risk** | 0.882 | 0.778 | 0.789 | 0.875 |
| **Low Risk** | 0.778 | 0.882 | 0.875 | 0.789 |

### Example 7.15: "Carc vs HR" - 40recursive feature extr >SVM 85%

The following markers were identified according to this example:
**List 17:** ABCF1, ASNA1, SCHIP1, CCDC94, CCDC94, CELSR1, CLEC3B, COBRA1, ECHS1, EDARADD, ETS1, FAM114A2, KIAA1109, FAM65A, FLNA, GMIP, INTS1, IRF3, KDM3B, LARP1, METTL3, PBXIP1, PRKD2, PSMB5, QTRT1, RPL26, RSL1D1, RSL1D1, SCHIP1, SHF, SPINT1, SPTBN1, SPTBN1, UBE2Q1, WDR73.

Then the "recursive feature" strategy for class prediction of all Carc vs HR samples was used, and it was possible to very efficiently build a classifier for distinguishing classes. Using 40 recursive features on arrays, the CCVP, SVM and BCCVP predictor enabled correct classification of 85% of samples.

Recursive Feature Elimination method was used to select 40 genes. Repeated 1 times K-fold (K= 20) cross-validation method was used to compute mis-classification rate.

**Performance of classifiers during cross-validation.**

| | **Array id** | **Class label** | **Mean Number of genes in classifier** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Com-pound Covariate Predictor Correct?** |
|---|---|---|---|---|---|---|---|---|---|---|
| **Mean percent** | | | | **85** | **82** | **74** | **79** | **79** | **85** | **85** |
| **of correct classification:** | | | | | | | | | | |

**Performance of the Compound Covariate Predictor Classifier:**

| **Class** | **Sensitivity** | **Specificity** | **PPV** | **NPV** |
|---|---|---|---|---|
| **Carcinoma** | 0.941 | 0.765 | 0.8 | 0.929 |
| **High Risk** | 0.765 | 0.941 | 0.929 | 0.8 |

**Performance of the Support Vector Machine Classifier:**

| **Class** | **Sensitivity** | **Specificity** | **PPV** | **NPV** |
|---|---|---|---|---|
| **Carcinoma** | 0.882 | 0.824 | 0.833 | 0.875 |
| **High Risk** | 0.824 | 0.882 | 0.875 | 0.833 |

**Performance of the Bayesian Compound Covariate Classifier:**

| **Class** | **Sensitivity** | **Specificity** | **PPV** | **NPV** |
|---|---|---|---|---|
| **Carcinoma** | 0.941 | 0.706 | 0.762 | 0.923 |
| **High Risk** | 0.706 | 0.941 | 0.923 | 0.762 |

### Example 7.16: "Carc vs HR" / 20 greedy pairs > SVM=85%

The following markers were identified according to this example:
**List 18:** ADRBK1, ATCAY, C10orf118, CELSR1, L1CAM, CYB5R3, G6PD, GMIP, GTF2I, HNRNPA1, SNAPIN, LRSAM1, MUM1, NASP, NFKBID, NR4A1, PGK1, PHF19, RBM4, RPL27, RSL1D1, RSL1D1, ACO2 (includes EG:11429), SF3B1, TALDO1, TCEA2, THBS1, TPM3, ZWINT, TSPAN7, ZMIZ.

Again using the "greed pairs" strategy for class prediction of Carc vs HR samples, 40 features on arrays, enabled correct classification of 85% of samples by the SVM classifier.

Greedy pairs algorithm was used to select 20 pairs of genes. Repeated 1 times K-fold (K= 20) cross-validation method was used to compute mis-classification rate.

**Performance of classifiers during cross-validation.**

| | **Array id** | **Class la-bel** | **Mean Number of genes in classifier** | **Compound Covariate Predictor Correct?** | **Diagonal Linear Discriminant Analysis Correct?** | **1-Nearest Neighbor** | **3-Nearest Neighbors Correct?** | **Nearest Centroid Correct?** | **Support Vector Machines Correct?** | **Bayesian Compound Covariate Predictor Correct?** |
|---|---|---|---|---|---|---|---|---|---|---|
| **Mean percent of correct classification:** | | | | **71** | **74** | **79** | **76** | **76** | **85** | **75** |

**Performance of the Support Vector Machine Classifier:**

| **Class** | **Sensitivity** | **Specificity** | **PPV** | **NPV** |
|---|---|---|---|---|
| **Carcinoma** | 0.882 | 0.824 | 0.833 | 0.875 |
| **High Risk** | 0.824 | 0.882 | 0.875 | 0.833 |

### DWD transformed data

In analogy to the "Class-prediction analyses" and examples depicted after Quantil-Normalization (QNORM) of protein-chip data, DWD-transformed data were similarly analyzed.

**Example 7.17:** Healthy vs diseased samples (Carc-HR-LR) comprising carcinoma and patients with polyps could be distinguished by a *1-Nearest Neighbour* classifier at 83% correct classification using 25 *greedy pairs* for feature selection.

The following markers were identified according to this example:
**List 19:** ADH5 (includes EG:11532), ALDOA, ARHGEF1, ATXN10, BIN3, C17orf28, TMEM98, C17orf90, AFP, AFP, CDK16, COL3A1, CYC1, DCTPP1, DEF8, EIF4A2, RPA1, TACC2, ACTL6B, FAM160A2, LAMB3, LMTK2, LRRC4C, LTBP3, MTCH2, PAPLN, PDIA3, PDP1, PI4KA, PKM2, PLAA, PLCG1, PPP1R2, RBM4, RPL37A, SLC4A3, SPRY1, MGAT4B, ST3GAL3, TSTA3, UROD, USP7, PAICS, VASP, VPS18, ZNF668.

**Example 7.18:** Healthy vs diseased samples (Carc-HR-LR) comprising carcinoma and patients with polyps could be distinguished by a *Nearest Centroid* classifier at 83% correct classification using 40 *greedy pairs* for feature selection.

The following markers were identified according to this example:
**List 20:** ACADVL, ADH5 (includes EG:11532), AGFG1, ALDOA, ARHGEF1, ASB13, ATXN10, BIN3, C17orf28, TMEM98, C17orf90, AFP, AFP, CCNI, CDK16, COL3A1, CPLX1, CPNE6, CTDP1, CYC1, DCTPP1, DEF8, EIF4A2, RPA1, EPRS, TACC2, ACTL6B, FAM160A2, JMJD4, BTBD6, IMP4, LAMB3, LCP1, LMO7, LMTK2, LRRC4C, LTBP3, MRPL47, MRPS11, MTCH2, MTSS1L, NARFL, NBEAL2, PAPLN, PDIA3, PDP1, PI4KA, PKM2, PLAA, PLCG1, PLXND1, PPP1R2, PPP4R1, RBM4, RBM4, RPL26, RPL37A, SERPINA1, SF3B4, SLC4A3, SLC25A29, SNRNP40, SPRY1, SSRP1, MGAT4B, TMUB2, ST3GAL3, TSTA3, UROD, USP7, PAICS, VASP, VPS18, YARS, ZNF410, ZNF668.

**Example 7.19:** Controls vs Carcinomas of a subset of 34 samples conducted in the same analyses run could be distinguished by a Compound Covariate, Support Vector Machines, and *Bayesian Compound Covariate Predictor* classifier at 94% correct classification using 25 *greedy pairs* for feature selection.

The following markers were identified according to this example:
**List 21:** ACAA2, ASB13, ATXN2, CAMK1D, CCL28, CKM, COL3A1, CUL1, CUL9, DCTPP1, DDX54, DOCK10, EEF1A1, TACC2, GSTP1, HMG20B, HMGN2, HNRNPK, INTS7, ISG15, LIMD2, LMNA, LPCAT1, MACF1, NASP, KPNA2, PAPLN, PBRM1, PDZD4, PHACTR3, PLEKHA5, PLXND1, PRDM2, RNF220, SGSH, SPRY1, ST6GALNAC1, TSTA3, TTYH3, TUBGCP2, UBB, ZNF174, ZNF410.

**Example 7.20:** Controls vs Carcinomas of the entire study samples could be distinguished by a Compound Covariate *Predictor* classifier at 87% correct classification using 25 *greedy pairs* for feature selection.

The following markers were identified according to this example:
**List 22:** ACTL6B, ARHGEF10L, ARHGEF17, ATXN2, TMEM98, C17orf90, AFP, AFP, COL3A1, CPLX1, CYC1, DCTPP1, DOCK9, EIF4A2, ERBB3 (includes EG:13867), TACC2, ACTL6B, PLEKHO1, FAM204A, GEMIN2, GPSM1, HAUS4, IMP4, ISG15, LIMD2, LRRC4C, MACF1, MTCH2, NBEAL2, KPNA2, PAPLN, PKM2, PLAA, PLCG1, PLXND1, POTEE/POTEF, PPP4R1, PSMB4, REV3L, RPL37A, RSL1D1, KLHL23/PHOSPHO2-KLHL23, RTN4 (includes EG:57142), SLC4A3, SPRY1, TMC8, TRIOBP, TSTA3, TUBB3, UROD, PAICS, VPS18, ZNF410.

**Example 7.21:** Controls vs Carcinomas of the entire study samples could be distinguished by a Compound Covariate *Predictor* classifier at 89% correct classification using 15 *greedy pairs* for feature selection.

The following markers were identified according to this example:
**List 23:** ACTL6B, ATXN2, TMEM98, C17orf90, AFP, AFP, COL3A1, CPLX1, DCTPP1, ERBB3 (includes EG:13867), TACC2, ACTL6B, GEMIN2, GPSM1, ISG15, LRRC4C, MACF1, MTCH2, NBEAL2, KPNA2, PAPLN, PLCG1, REV3L, RPL37A, RSL1D1, TRIOBP, TSTA3, UROD, PAICS, VPS18, ZNF410.

**Example 7.22:** Controls vs Carcinomas of the entire study samples could be distinguished by a *Support Vector Machines* classifier at 83% correct classification using *RECURSIVE feature extraction (n=40)* for feature selection.

The following markers were identified according to this example:
**List 24:** ACTL6B, AFTPH, AKAP9, ARHGEF1, ASNA1, CCL28, CDH2, CSK, DCTPP1, DDX3Y, DNAJC10, EML2, GGA1 (includes EG:106039), GP1BB, HADHA, HMGN2, ISG15, KIAA0368, LRSAM1, MCM3AP, MTCH2, KPNA2, PKD1L1, POGZ, PRMT1, PRPF4B, QTRT1, SAMSN1, GRK6, SERBP1, TRAPPC3, ST3GAL3, YLPM1, YWHAZ.

**Example 7.23:** "Controls and low risk poly-patients" vs "Carcinomas and high risk polyps" of the entire study samples could be distinguished by a *1-Nearest neighbor* classifier (143 features) at 76% correct classification using *a single-gene p-value of* p<0.005 *as cut-off* for feature selection.

The following markers were identified according to this example:
**List 25:** ABCE1, ACAA2, ACTL6B, ADH5 (includes EG:11532), AKT2, IGSF9, ANXA6, ARHGEF10L, ARHGEF25, ASB13, ASPSCR1, AXIN1, PECAM1, C6orf136, CCL28, CERCAM, CLDN5, CLU, CPE (includes EG:12876), DBI, DCAF11, DCTPP1, DDR1, DDX27, DENR, DHX58, DLG5, EPC1, ACTL6B, PLEKHO1, EXOSC4, FAM213A, FBLL1, FBRS, FKBP15, FLII, FN1, GOSR1, GTF2B, HAPLN3, HARS, HAUS4, HEBP2, HLA-C, HNRNPK, HNRNPR, HNRPLL, HSPA8, INPP5K, INPPL1, INTS1, INTS1, ISG15, KDM4A, LAMB1, LCP1, LOC100132116, LRIG1, LRRC4C, LTBP3, LYSMD2, MAF1 (includes EG:315093), MRPL38 (includes EG:303685), MTCH1, MTCH2, RPL17, MYO5A, NARF, NBEAL2, NBR1, NDUFB10, KPNA2, OTUD1, PABPC1, PAPLN, PI4KA, PIK3IP1, PKD1L1, PKP3, PLXNA3, PLXND1, POMT1, PPP1R13B, PPP4R1, PRDX5, PRRT1, PSKH1, PSMB4, REC8 (includes EG:290227), RGS19, RHBDD2, RPL4, RNF40, RPL13, RPL17, RPL22, RPN1, SAT2, SERPINH1, SGSH, SH3BP2, SLC25A20, SMCHD1, STMN4, TBC1D9B, TBCB, TIAM2, TMC8, TMCC2, TPM3, ZWINT, TRAPPC4, TSTA3, UBE2N, USP48, VARS, VPS18, ZFP14, ZNF133, ZNF410, ZNF668, ZNF672.

**Example 7.24:** "Controls and low risk poly-patients" vs "Carcinomas and high risk polyps" of the entire study samples could be could be distinguished by a *Diagonal Discriminant Analyses Predictor* classifier at 81% correct classification using 25 *greedy pairs* for feature selection.

The following markers were identified according to this example:
**List 26:** ACAA2, ADH5 (includes EG:11532), ARHGEF10L, ASPSCR1, TMEM98, C17orf90, AFP, AFP, COX7A2L, DCTPP1, DENR, DLG5, EIF4A2, EPC1, ACTL6B, PLEKHO1, FLII, HLA-C, HNRNPA2B1, HNRNPR, INPP5K, ISG15, LRRC4C, LTBP3, MAF1 (includes EG:315093), MTCH2, KPNA2, PAPLN, LOC440354, PLXND1, RGS19, RNF40, RPL22, SPRY1, SRRM2, TBC1D9B, TMC8, TSTA3, TUBB3, PAICS, VPS18, ZNF410, ZNF668, ZNF672.

**Example 7.25:** Exemplifying the separation of all 4 different sample-classes, a "Binary tree" prediction analysis was conducted.

The following markers were identified according to this example:
**List 27, part a):** AARS, ABCA2, ACADVL, ACSL3, ACTB, ADCY1, ADH5 (includes EG:11532), AFTPH, AGFG1, AGPAT6, AHSG, AKR1C4, AKR1C4, AKT1, ALDOA, ANAPC5, IGSF9, ANTXR2, ANXA6, AP2A1, BTBD7, APBA3, APP, ARF4, ARF5, GDAP1L1, ASB13, ASMTL, ATP5D, ATXN10, ATXN10, BCS1L, BRD3, BTBD11, C10orf76, C17orf90, AFP, C18orf21, PECAM1, CALR, CAPN2, CARM1, NDUFB7, CCDC109B, CCDC136, CCL28, CDC37, CDK16, CDR1, CHD1L, CHGA, COPS3, COX7A2L, CPNE1, CPNE6, CSNK2B, CTSD, DCTN3, DCTPP1, DDB1, DDR1, DDX23, DDX41, DDX56, DEF8, DENND3, ARHGEF40, DPP3, EEF1A1, EEF1A1, EEF1A1, EHMT2, EIF2B5, RPA1, EPHB6, EPN1, EPRS, TACC2, ESYT1, ARPC1A, FAM193A, ARPC1A, FAM193A, FAM32A, FLII, FLNA, GAL3ST4, GGA1 (includes EG:106039), GHITM, GOLGA4, GSK3A, GTPBP3, C10orf2, HES6, HLA-C, HMGA1, HMGCL, HMGN2, JMJD4, HSD17B4, HSP90AB1, IDH3B, IGHA1, KAT5, KAT7, KCTD13, KHSRP, KIF19, KIFAP3, LAMB3, LCMT1, LCP1, LDLR, LMTK2, LTBP3, MACF1, TCF19, MAP1S, MAPK3, MAPK7, MARS, MATR3, MEAF6, MGAT4B, MIIP, MLL4, MTCH1, RPL17, NARG2, NBEAL2, NDUFB10, NDUFS2, NES, NLRC5, NME2, NOL12, NOMO1 (includes others), NPDC1, NRBP2, NOA1, OGT, ALB, PAICS, PALM, PCDH9, PDIA3, PDP1, PHF8, PI4KA, PKM2, PLAA, PKM2, PLAA, PLCE1, PLCG1, PLXND1, PODXL2, POGZ, POMT1, PPM1F, PPP1R2, PRDX1, PRKCZ, PRRT1, PSAT1, PSMA2, PSMD6, PSMD8, PSME1, PTCHD2, ARHGDIA, PTOV1, PTPRS, BAD, HAPLN3, RAI1, RBM15, RBM4, RBM4, RBM4, RHBDD2, RNF216, RNF220, RPL26, RPL27, RPL37A, RPL4, RPS21, RPS27A, KLHL23/PHOSPHO2-KLHL23, RTN4 (includes EG:57142), SCAF8, SCOC, SFXN1, SLC4A3, DARS, SH3GL1, SLA, SLC9A3R1, SMUG1, SNIP1, SRA1, SRA1, SRSF1, SSBP2, SSBP2, ST6GALNAC1, STIM2, STK25, STX6, SUV420H1, TADA2B, TARS, TBC1D7, MGAT4B, TERF2IP, HSPA5, TLE1, TMC8, TMED8, PXN, TNFRSF25, DSN1 (includes EG:100002916), TOE1, ST3GAL3, TRIM27, TTYH1, GPX4, TUBA1B, TUBA1B, TUBA1B, TUBB, TUBB, TUBGCP2, TWF2, UBB, UBE2N, UBXN1, UROD, TUBA1B, USP7, PAICS, VPS26A, WDR35, WDR6, WSB2, YARS, ZNF133, ZNF174, ZNF300, ZNF408, ZNF423, ZNF605, ZNF668. **List 27, part b):** ABCE1, ACAA2, ACAT2, AFMID, AKAP17A, AKT2, ALAD, ALB, AP2A1, BTBD7, AP2A1, BTBD7, APRT, APRT, ZNF259, ARHGAP44, ARHGEF10L, ARHGEF25, ARRB2, CBR1, CCDC88B, CCL28, CLU, CORO7, CRABP1, CSK, CUL9, DCAF11, DCTPP1, DDIT4, DDR1, DDX19B, DDX27, DENR, DLG5, DPP9, DUS1L, DVL1, EEF1A1, EEF1A1, ELP2, EPC1, FCGBP, FLII, GLUL, GMPPB, HIPK3, HNRNPA0, HNRNPH1, HSPA8, IL2RG, IL6ST, INPP5K, INTS1, ISG15, ITGAL, LMAN1, LONP1, LRIG1, MAF1 (includes EG:315093), MCM3AP, MED4 (includes EG:29079), MGAT4B, MRPL27 (includes EG:287635), MTA2, MYH9, MYO5A, NDRG1, NDUFA13, NDUFB10, NISCH, KPNA2, OTUB1, PDE4D, PECAM1, PERI, PJA1, PLEKHG2, PLXNA3, PLXND1, POR, PPP1R13B, PRPF6, PRR3, PSKH1, RABEPK, HAPLN3, RBM26, SULT1A3/SULT1A4, RNF40, RPL17, RPL22, RPS6KA1 (includes EG:20111), RTKN, SEMA3F, SEPT1, GRK6, SERBP1, SERPINB9, SETD2, SGSH, SH3BP2, SLC9A3R1, SMCHD1, SNF8, SPRN, SRA1, SYS1 (includes EG:336339), TAX1BP1, TBC1D9B, TBCB, TMC8, TRPS1, TTC28, TUBB6, VARS, WDR63, XYLT1, ZNF133, ZNF408, ZNF672, ZNF784.

As illustrated in that example the 1st classification node separates "Controls" from all the remaining classes using the markers of list 27, part a) as classifier at a "Mis-classification rate" (MCR) of 7.1%, then "Low Risk" polyp-patients are distinguished from "Carcinoma & High Risk" by the markers of list 27, part b) (MCR=15.4%).

The markers of List 27, part a) can be used independently from the markers of list 27 part b) to distinguish health controls from the combined group of cancer, LR polyps and HR polyps. Markers of list 27 part b) can be used to distinguish the indicated groups of classes (medical indications; in this case LR vs. cancer plus HR) but work best if the groups have been preselected by using the distinguishing markers of list 27) in a previous step to remove the healthy controls from the question to be solved.

Binary Tree Classification algorithm was used. Feature selection was based on the univariate significance level (alpha = 0.001). The support vector machine classifier was used for class prediction. There were 2 nodes in the classification tree. 10-fold cross-validation was performed.

Cross-validation error rates for a fixed tree structure shown below.

| **Node** | **Group 1 Classes** | **Group 2 Classes** | **Mis-classification rate (%)** |
|---|---|---|---|
| **1** | Carcinoma, High Risk, Low Risk | Control | 7.1 |
| **2** | Carcinoma, High Risk | Low Risk | 15.4 |

**Example 7.26:** "Controls" vs "low risk poly-patients" of the entire study samples could be could be distinguished by a *Nearest Centroid Predictor* classifier at 89% correct classification using 25 *greedy pairs* for feature selection.

The following markers were identified according to this example:
**List 28:** AHSG, AKR1B1, BTBD11, CANX, CPNE1, DHX8, EHD4, RPA1, FBXO21, C10orf2, HMGCL, IKBKAP, ITGAL, LMTK2, MAPKAP1, MARS, MATR3, MUC2, NDUFS2, PDP1, PSMD6, PTPRS, BAD, RPL17, RPL37A, SEPT1, SLC4A3, STK17A, SUV420H1, TBC1D7, TUBA1B, TUBB6, WDR1.

**Example 7.27:** "Low risk vs high risk polyp-patients" of the entire study samples could be could be distinguished by a *Support Vector Machines* classifier at 86% correct classification using *25 greedy pairs* for feature selection.

The following markers were identified according to this example:
**List 29:** ABCE1, AKAP17A, AP2A1, BTBD7, CELSR1, CYB5R3, DLG5, DOC2B, EXOSC4, FCGBP, FLII, FNBP4, GTF2B, HERPUD1, HIST1H2AC, HNRNPM, HNRNPR, HSPA8, IK, MED4 (includes EG:29079), NBPF11 (includes others), PECAM1, PLEKHG2, PRDX5, PREP, REEP2, RNF10, RPL17, RPL22, RTKN, SEPT1, GRK6, SERBP1, SERBP1, NAP1L1, SYS1 (includes EG:336339), TBC1D9B, TBCB, XYLT1, ZNF672.

**Example 7.28:** "Carcinoma vs high risk polyp-patients" of the entire study samples could be could be distinguished by a *1-Nearest Neighbor* classifier at 68% correct classification using 25 *greedy pairs* for feature selection.

The following markers were identified according to this example:
**List 30:** ARHGEF10L, ATXN2, BCAM, C10orf118, C1orf174, C20orf20, CDK16, CELSR1, CLTA, CPSF1, L1CAM, ELP3, HMGN2, FASN, FBN3, G6PD, GNB1, SNAPIN, IDOl, LRSAM1, MUM1, NASP, PDE2A, PDZD4, PIK3IP1, POLR2E, PTPRF, PXN, REEP2, RPL27, RSL1D1, SF3B1, SLC25A6, SMTN, SRA1, TESK1, THBS3, TIPARP-AS1, TNXB, TPM3, ZWINT, TSPAN7, UBE2Q1, ZMIZ2, ZNF768.

### Example 7.29:

"Carcinoma vs high risk polyp-patients" of the entire study samples could be could be distinguished by a *Support Vector Machines* classifier at 68% correct classification using *recursive feature extraction (n=40)* for feature selection.

The following markers were identified according to this example:
**List 31:** ACSS1, ASNA1, SCHIP1, CCDC94, CDH2, CELSR1, CHN2, DUSP4, ECHS1, EDARADD, EID1, FAM114A2, GLRX3, INTS1, NAP1L4, PFKL, PKM2, PLAA, PLEC, PRKD2, PRPF31, PSMB5, QTRT1, RABGGTA, SCHIP1, ACO2 (includes EG:11429), SERPINB9, SF3B1, SHF, SLC4A2, SPTBN1, SPTBN1, TESK1, TUBGCP6, UBE2Q1, USP5, ZNF358.

### Example 8: Random selection of subsets of classifiers

To exemplify the diagnostic potential of subsets from classifiers elucidated here, 10 markers were randomly chosen: Set A: UROD, ERCC5, RBM4, TCEAL2, PLXND1, ALDOA, LCP1, TMUB2, CTDP1, RPL3 (UniGene ID: Hs.575313); Set B: EXOC1, SGTA, PPP1R2, SERPINA1, YARS, ZNF410, CTDP1, SLC25A29, COL3A1, DCTPP1; from e.g. the 80 classifiers markers depicted after DWD transformation for distinguishing patients with carcinomas and polyps (Carc & HR & LR; n=84) versus Controls (n=50) (contrast 1). Then class prediction analysis was conducted and elucidated with the random sets A) 82%, and B) 75% correct classification by the Nearest Centroid Classifier (NCentr); - originally the NCentr classifier derived from the 80 genes enabled 83% correct classification. This confirms that classifiers from the present gene-lists have high potential for diagnostics, and that subsets of the classifiers panels as well as single classifiers are of high potential value for diagnostics.

To reconfirm this findings for the QNORM set of classifiers (the 2nd applied data normalization strategy in addition to DWD), the same 2 subsets were retested, of 10 random-classifiers on the QNORM data set. Class prediction analysis for distinguishing patients with carcinomas and polyps (Carc_HR_LR; n=84) versus Controls (n=50) (contrast 1) was again conducted. Random set A) enabled 83% correct classification, and random set B) 75% correct classification by the Nearest Centroid Classifier (NCentr); originally these 2 sets of 10 markers were only presented by some markers in the QNROM derived classifier.

Therefore subsets of the classifiers panels as well as single classifiers would be useful for classification and diagnostics, independent from initial normalization strategies upon which classifiers were generated.

Again for exemplification of the value of subsets of classifier markers from the panels defined in that application, the 50 classifier genes for distinguishing Cancer vs Controls were selected and randomly chosen subsets of 2-49 markers (x-axis) were selected 1000-times. Then the classification success obtained by that subsets using the nearest centroid classifier algorithm was calculated and median % misclassification is depicted on the y-axis (Fig 2).

The 80 classifier markers for distinguishing Disease (Cancer & polyps) vs Controls were selected and randomly chosen subsets of 2-79 markers (x-axis) were selected 1000-times. Then the classification success obtained by that subset using the nearest centroid classifier algorithm was calculated and median % mis-classification is depicted on the y-axis (Fig 3). Thus even single classifier markers are capable of correct classification (80% correct for set A) - corresponds to a median classification-error of 20% - Fig 3; and e.g. 70% correct for set B) corresponds to a median classification-error of 30% - Fig 3).

## Claims

1. In vitro method of diagnosing colon cancer or the risk of colon cancer in a patient by detecting autoantibodies against the marker protein ArsA Arsenite Transporter, ATP-Binding, Homolog 1 (ASNA1) and autoantibodies against at least 1 of the marker proteins selected from the markers Ecm29 proteasome adaptor and scaffold (KIAA0368 also known as ECPAS), queuine tRNA-ribosyltransferase catalytic subunit 1 (QTRT1), SAM domain, SH3 domain and nuclear localization signals 1 (SAMSN1), glycoprotein Ib platelet subunit beta (GP1BB), actin like 6B (ACTL6B), aftiphilin (AFTPH), A-kinase anchoring protein 9 (AKAP9), Rho guanine nucleotide exchange factor 1 (ARHGEF1), C-C motif chemokine ligand 28 (CCL28), cadherin 2 (CDH2), C-terminal Src kinase (CSK), dCTP pyrophosphatase 1 (DCTPP1), DEAD-box helicase 3 Y-linked (DDX3Y), DnaJ heat shock protein family (Hsp40) member C10 (DNAJC10), EMAP like 2 (EML2), golgi associated, gamma adaptin ear containing, ARF binding protein 1 (GGA1), hydroxyacyl-CoA dehydrogenase trifunctional multienzyme complex subunit alpha (HADHA), high mobility group nucleosomal binding domain 2 (HMGN2), ISG15 ubiquitin like modifier (ISG15), leucine rich repeat and sterile alpha motif containing 1 (LRSAM1), minichromosome maintenance complex component 3 associated protein (MCM3AP), mitochondrial carrier 2 (MTCH2), karyopherin subunit alpha 2 (KPNA2), polycystin 1 like 1, transient receptor potential channel interacting (PKD1L1), pogo transposable element derived with ZNF domain (POGZ), protein arginine methyltransferase 1 (PRMT1), pre-mRNA processing factor 4B (PRPF4B), G protein-coupled receptor kinase 6 (GRK6), SERPINE1 mRNA binding protein 1 (SERBP1), trafficking protein particle complex 3 (TRAPPC3), ST3 beta-galactoside alpha-2,3-sialyltransferase 3 (ST3GAL3), YLP motif containing 1 (YLPM1), tyrosine 3-monooxygenase/tryptophan 5-monooxygenase activation protein zeta (YWHAZ) in a patient comprising the step of detecting the autoantibodies against said marker proteins in a sample that has been taken from the patient, wherein the detection of said autoantibodies of the detecting step indicate colon cancer or the risk of colon cancer.

2. The method of claim 1 comprising diagnosing cancer or pre-cancerous polyps, preferably wherein said precancerous polyps are distinguished between high-risk polyps and low-risk polyps, wherein said high-risk polyps comprise adenomatous villous, adenomatous tubulovillous, or co-occurrence of adenomatous tubular with tubulovillous polyps.

3. The method of claim 1 or 2 comprising distinguishing between healthy conditions vs. cancer.

4. The method of any one of claims 1 to 3, wherein the step of detecting autoantibodies against said marker proteins comprises comparing said detection signal with detection signals of a healthy control and comparing said detection signals, wherein an increase in the detection signal indicates colon cancer or said risk of colon cancer.

5. The method of any one of claims 1 to 4, wherein the step of detecting autoantibodies against said marker proteins comprises comparing said detection signal with detection signals of one or more known control samples of conditions selected from cancer, pre-cancerous polyps, and/or a healthy control, wherein the control signals are used to obtain a marker dependent signal pattern as indication classifier and the marker dependent signals of the patient is compared with and/or fitted onto said pattern thereby obtaining information of the diagnosed condition.

6. The method of any one of claims 1 to 5, wherein the step of detecting autoantibodies against said marker proteins comprises comparing said detection signal with detection signals of a cancerous or pre-cancerous polyp control and comparing said detection signals, wherein a detection signal from the sample of the a patient in amplitude of at least 60%, preferably at least 80%, of the cancerous control indicates colon cancer or said risk of colon cancer.

7. The method of any one of claims 1 to 5, wherein the step of detecting autoantibodies against said marker proteins comprises comparing said detection signal with detection signals of a cancerous or pre-cancerous polyp control and comparing said detection signals, wherein a detection signal in at least 60% of the used markers indicates colon cancer or said risk of colon cancer.

8. The method of claim 7, wherein a detection signal in at least 75% of the used markers indicates colon cancer or said risk of colon cancer.

9. Use of a kit suitable to detect autoantibodies against any marker combination as defined in claim 1, wherein the kit comprises diagnostic agents that comprise marker proteins selected from ArsA Arsenite Transporter, ATP-Binding, Homolog 1 (ASNA1) and at least 1 of the marker proteins selected from the markers Ecm29 proteasome adaptor and scaffold (KIAA0368 also known as ECPAS), queuine tRNA-ribosyltransferase catalytic subunit 1 (QTRT1), SAM domain, SH3 domain and nuclear localization sig-nals 1 (SAMSN1), glycoprotein Ib platelet subunit beta (GP1BB), actin like 6B (ACTL6B), aftiphilin (AFTPH), A-kinase anchoring protein 9 (AKAP9), Rho guanine nucleotide exchange factor 1 (ARHGEF1), C-C motif chemokine ligand 28 (CCL28), cadherin 2 (CDH2), C-terminal Src kinase (CSK), dCTP pyrophos-phatase 1 (DCTPP1), DEAD-box helicase 3 Y-linked (DDX3Y), DnaJ heat shock protein family (Hsp40) member C10 (DNAJC10), EMAP like 2 (EML2), golgi associated, gamma adaptin ear containing, ARF binding protein 1 (GGA1), hydroxyacyl-CoA dehydrogenase trifunctional multienzyme complex subunit alpha (HADHA), high mobility group nucleosomal binding domain 2 (HMGN2), ISG15 ubiquitin like modifier (ISG15), leucine rich repeat and ster-ile alpha motif containing 1 (LRSAM1), minichromosome mainte-nance complex component 3 associated protein (MCM3AP), mito-chondrial carrier 2 (MTCH2), karyopherin subunit alpha 2 (KPNA2), polycystin 1 like 1, transient receptor potential channel interacting (PKD1L1), pogo transposable element de-rived with ZNF domain (POGZ), protein arginine methyltransfer-ase 1 (PRMT1), pre-mRNA processing factor 4B (PRPF4B), G pro-tein-coupled receptor kinase 6 (GRK6), SERPINE1 mRNA binding protein 1 (SERBP1), trafficking protein particle complex 3 (TRAPPC3), ST3 beta-galactoside alpha-2,3-sialyltransferase 3 (ST3GAL3), YLP motif containing 1 (YLPM1), tyrosine 3-monooxygenase/tryptophan 5-monooxygenase activation protein zeta (YWHAZ) or antigenic fragments thereof suitable to bind the autoantibodies in a sample; in a method of any one of claims 1 to 8.

10. The use of claim 9, the kit further comprising signal data for control samples with known conditions selected from cancer or a pre-cancerous polyp, and/or of healthy controls.

11. The use of claim 9 or 10, the kit further comprising calibration or training data for analysing said markers provided in the kit for diagnosing colon cancer or distinguishing conditions selected from healthy conditions, cancer, or the pre-cancerous polyps.

12. The use of any one of claims 9 to 11, wherein said diagnostic agents are immobilized on a solid support.

13. The use of any one of claims 9 to 12, the kit comprising at most 3000 diagnostic agents.

14. The use of any one of claim 9 to 12, the kit comprising at most 2000 diagnostic agents.

15. The use of claim 14, the kit comprising at most 1000 diagnostic agents.

16. The use of claim 15, the kit comprising at most 200 diagnostic agents.

## Patentansprüche

1. In vitro-Verfahren zur Diagnose von Darmkrebs oder des Risikos von Darmkrebs bei einem Patienten durch Nachweisen von Autoantikörpern gegen das Markerprotein ArsA Arsenite Transporter, ATP-Binding, Homolog 1 (ASNA1) und von Autoantikörpern gegen mindestens eines der Markerproteine, ausgewählt aus den Markern ECM29 Proteasome Adaptor and Scaffold (KIAA0368, auch bekannt als ECPAS), katalytische Untereinheit der Queuin-tRNA-Ribosyltransferase 1 (QTRT1), SAM-Domäne, SH3-Domäne und nukleäre Lokalisationssignale 1 (SAMSN1), Beta-Untereinheit des Plättchen-Glykoproteins Ib (GP1BB), Actin like 6B (ACTL6B), Aftiphilin (AFTPH), A-Kinase-Ankerprotein 9 (AKAP9), Rho-Guaninnukleotid-Austauschfaktor 1 (ARHGEF1), Chemokin (C-C-Motiv) -Ligand 28 (CCL28), Cadherin 2 (CDH2), C-terminale Src-Kinase (CSK), dCTP-Pyrophosphatase 1 (DCTPP1), DEAD-Box Helicase 3 Y-linked (DDX3Y), DnaJ-Familie der Hitzeschockproteine (Hsp40) Mitglied C10 (DNAJC10), EMAP like 2 (EML2), Golgi-assoziiertes, gamma-Adaptin-Ear-enthaltendes ARFbindendes Protein 1 (GGA1), Alpha-Untereinheit des trifunktionellen Multienzymkomplexes Hydroxyacyl-CoA-Dehydrogenase (HADHA), High Mobility Group Nucleosomal Binding Domain 2 (HMGN2), ISG15 Ubiquitin-ähnlicher Modifikator (ISG15), Leucin-reiche Wiederholung und steriles alpha-Motiv enthaltendes Protein 1 (LRSAM1), Minichromosome Maintenance Complex Component 3 associated Protein (MCM3AP), mitochondrialer Träger 2 (MTCH2), Karyopherin Alpha-2 Untereinheit (KPNA2), mit transientem Rezeptorpotenzialkanal interagierendes Polycystin 1-like-1 (PKD1L1), Pogo transposable element derived with ZNF domain (POGZ), Protein-Arginin-Methyltransferase 1 (PRMT1), prä-mRNA Prozessierungsfaktor 4B (PRPF4B), G-Protein-gekoppelte Rezeptorkinase 6 (GRK6), SERPINE1 mRNA-Bindungsprotein 1 (SERBP1), Trafficking Protein Particle Complex 3 (TRAPPC3), ST3 Beta-Galactosid Alpha-2,3-Sialyltransferase 3 (ST3GAL3), YLP-Motiv-enthaltendes Protein 1 (YLPM1), Tyrosin 3-Monooxygenase/Tryptophan 5-Monooxygenase Aktivierungsprotein zeta (YWHAZ), bei einem Patienten, umfassend den Schritt des Nachweisens der Autoantikörper gegen diese Markerproteine in einer Probe, die dem Patienten entnommen worden ist, wobei der Nachweis dieser Autoantikörper aus dem Detektierschritt Darmkrebs oder das Risiko von Darmkrebs anzeigt.

2. Verfahren nach Anspruch 1, umfassend das Diagnostizieren von Krebs oder präkanzerösen Polypen, vorzugsweise wobei diese präkanzerösen Polypen zwischen Hochrisiko-Polypen und Niedrigrisiko-Polypen unterschieden werden, wobei diese Hochrisiko-Polypen adenomatöse villöse, adenomatöse tubulovillöse oder gleichzeitiges Vorkommen von adenomatösen tubulären und tubulovillösen Polypen umfassen.

3. Verfahren nach Anspruch 1 oder 2, umfassend das Unterscheiden zwischen gesunden Bedingungen gegenüber Krebs.

4. Verfahren nach einem der Ansprüche 1 bis 3, wobei der Schritt des Nachweisens von Autoantikörpern gegen besagte Markerproteine das Vergleichen des Detektionssignals mit Detektionssignalen von einer gesunden Kontrolle und Vergleichen dieser Detektionssignale umfasst, wobei ein Anstieg im Detektionssignal Darmkrebs oder das Risiko von Darmkrebs anzeigt.

5. Verfahren nach einem der Ansprüche 1 bis 4, wobei der Schritt des Nachweisens von Autoantikörpern gegen besagte Markerproteine das Vergleichen des Detektionssignals mit Detektionssignalen von einer oder mehreren bekannten Kontrollproben von Bedingungen, ausgewählt aus Krebs, präkanzerösen Polypen und/oder einer gesunden Kontrolle umfasst, wobei die Kontrollsignale verwendet werden, um ein markerabhängiges Signalmuster als Indikationsklassifizierer zu erhalten, und die markerabhängigen Signale von dem Patienten verglichen werden mit dem Muster und/oder eingepasst werden auf dieses Muster, wodurch Informationen zu der diagnostizierten Bedingung erhalten werden.

6. Verfahren nach einem der Ansprüche 1 bis 5, wobei der Schritt des Nachweisens von Autoantikörpern gegen besagte Markerproteine das Vergleichen des Detektionssignals mit Detektionssignalen von einer kanzerösen oder präkanzerösen Polyp-Kontrolle und Vergleichen der Detektionssignale umfasst, wobei ein Detektionssignal von der Probe des Patienten in einer Signalstärke von mindestens 60 %, vorzugsweise mindestens 80 %, der kanzerösen Kontrolle Darmkrebs oder das Risiko von Darmkrebs anzeigt.

7. Verfahren nach einem der Ansprüche 1 bis 5, wobei der Schritt des Nachweisens von Autoantikörpern gegen besagte Markerproteine das Vergleichen des Detektionssignals mit Detektionssignalen von einer kanzerösen oder präkanzerösen Polyp-Kontrolle und Vergleichen der Detektionssignale umfasst, wobei ein Detektionssignal bei mindestens 60 % der verwendeten Marker Darmkrebs oder das Risiko von Darmkrebs anzeigt.

8. Verfahren nach Anspruch 7, wobei ein Detektionssignal bei mindestens 75 % der verwendeten Marker Darmkrebs oder das Risiko von Darmkrebs anzeigt.

9. Verwendung eines Kits, das geeignet ist, Autoantikörper gegen jede Markerkombination, wie in Anspruch 1 definiert, nachzuweisen, wobei das Kit Diagnostika umfasst, die Markerproteine, ausgewählt aus ArsA Arsenite Transporter, ATP-Binding, Homolog 1 (ASNA1) und mindestens einem von den Markerproteinen, ausgewählt aus den Markern ECM29 Proteasome Adaptor and Scaffold (KIAA0368, auch bekannt als ECPAS), katalytische Untereinheit der Queuin-tRNA-Ribosyltransferase 1 (QTRT1), SAM-Domäne, SH3-Domäne und nukleäre Lokalisationssignale 1 (SAMSN1), Beta-Untereinheit des Plättchen-Glykoproteins Ib (GP1BB), Actin like 6B (ACTL6B), Aftiphilin (AFTPH), A-Kinase-Ankerprotein 9 (AKAP9), Rho-Guaninnukleotid-Austauschfaktor 1 (ARHGEF1), Chemokin (C-C-Motiv) -Ligand 28 (CCL28), Cadherin 2 (CDH2), C-terminale Src-Kinase (CSK), dCTP Pyrophosphatase 1 (DCTPP1), DEAD-Box Helicase 3 Y-linked (DDX3Y), DnaJ-Familie der Hitzeschockproteine (Hsp40) Mitglied C10 (DNAJC10), EMAP like 2 (EML2), Golgiassoziiertes, gamma-Adaptin-Ear-enthaltendes ARF-bindendes Protein 1 (GGA1), Alpha-Untereinheit des trifunktionellen Multienzymkomplexes Hydroxyacyl-CoA-Dehydrogenase (HADHA), High Mobility Group Nucleosomal Binding Domain 2 (HMGN2), ISG15 Ubiquitin-ähnlicher Modifikator (ISG15), Leucin-reiche Wiederholung und steriles alpha-Motiv enthaltendes Protein 1 (LRSAM1), Minichromosome Maintenance Complex Component 3 associated Protein (MCM3AP), mitochondrialer Träger 2 (MTCH2), Karyopherin Alpha-2 Untereinheit (KPNA2), mit transientem Rezeptorpotenzialkanal interagierendes Polycystin 1-like-1 (PKD1L1), Pogo transposable element derived with ZNF domain (POGZ), Protein-Arginin-Methyltransferase 1 (PRMT1), prä-mRNA Prozessierungsfaktor 4B (PRPF4B), G-Protein-gekoppelte Rezeptorkinase 6 (GRK6), SERPINE1 mRNA-Bindungsprotein 1 (SERBP1), Trafficking Protein Particle Complex 3 (TRAPPC3), ST3 Beta-Galactosid Alpha-2,3-Sialyltransferase 3 (ST3GAL3), YLP-Motiv-enthaltendes Protein 1 (YLPM1), Tyrosin 3-Monooxygenase/Tryptophan 5-Monooxygenase Aktivierungsprotein zeta (YWHAZ) oder antigene Fragmente davon, die zur Bindung an die Autoantikörper in einer Probe geeignet sind, umfassen; bei einem Verfahren nach einem der Ansprüche 1 bis 8.

10. Verwendung nach Anspruch 9, wobei das Kit außerdem Signaldaten für Kontrollproben mit bekannten Bedingungen, ausgewählt aus Krebs oder einem präkanzerösen Polypen und/oder von gesunden Kontrollen, umfasst.

11. Verwendung nach Anspruch 9 oder 10, wobei das Kit außerdem Kalibrations- oder Trainingsdaten zur Analyse der Marker, die in dem Kit zur Diagnose von Darmkrebs oder zur Unterscheidung von Bedingungen, ausgewählt aus gesunden Bedingungen, Krebs oder den präkanzerösen Polypen, bereitgestellt sind, umfasst.

12. Verwendung nach einem der Ansprüche 9 bis 11, wobei besagte Diagnostika an einem festen Träger immobilisiert sind.

13. Verwendung nach einem der Ansprüche 9 bis 12, wobei das Kit höchstens 3000 Diagnostika umfasst.

14. Verwendung nach einem der Ansprüche 9 bis 12, wobei das Kit höchstens 2000 Diagnostika umfasst.

15. Verwendung nach Anspruch 14, wobei das Kit höchstens 1000 Diagnostika umfasst.

16. Verwendung nach Anspruch 15, wobei das Kit höchstens 200 Diagnostika umfasst.

## Revendications

1. Procédé in vitro de diagnostic du cancer du côlon ou du risque de cancer du côlon chez un patient par détection d'auto-anticorps contre la protéine marqueur ArsA Arsenite Transporter, ATP-Binding, Homolog 1 (ASNA1) et d'auto-anticorps contre au moins 1 des protéines marqueurs sélectionnées parmi les marqueurs adaptateur et échafaudage du protéasome Ecm29 (KIAA0368, également appelé ECPAS), sous-unité catalytique 1 de queueine ARNt-ribosyltransférase (QTRT1), signaux de localisation nucléaire 1 du domaine SAM et du domaine SH3 (SAMSN1), glycoprotéine Ib sous-unité plaquettaire bêta (GP1BB), homologue à l'actine 6B (ACTL6B), af-tiphilin (AFTPH), protéine d'ancrage A-kinase 9 (AKAP9), facteur d'échange de nucléotides Rho guanine 1 (ARHGEF1), ligand de chimiokine motif CC 28 (CCL28), cadhérine 2 (CDH2), C-terminale de la kinase Src (CSK), dCTP pyrophosphatase 1 (DCTPP1), DEAD-box hélicase 3 Y-linked (DDX3Y), famille de protéines du choc thermique DnaJ (Hsp40) membre C10 (DNAJC10), EMAP like 2 (EML2), protéine de liaison ARF 1 associée à golgi, gamma adaptin ear (GGA1), sous-unité alpha du complexe multienzymatique trifonctionnel hydroxy-acyl-CoA déshydrogénase (HADHA), domaine de liaison nucléosomique du groupe à haute mobilité 2 (HMGN2), modificateur de type ubiquitine ISG15 (ISG15), répétition riche en leucine et motif alpha stérile contenant 1 (LRSAM1), protéine associée du composant 3 du complexe d'entretien des minichromosomes (MCM3AP), porteur mitochondrial 2 (MTCH2), sous-unité de caryophérine alpha 2 (KPNA2), polycystine 1 like 1, interagissant sur le canal potentiel du récepteur transitoire (PKD1L1), élément transposable pogo dérivé avec domaine ZNF (POGZ), protéine arginine méthyltransférase 1 (PRMT1), facteur de traitement pré-ARNm 4B (PRPF4B), récepteur kinase 6 couplé à la protéine G (GRK6), protéine de liaison à l'ARNm SERPINE1 1 (SERBP1), complexe de particules protéiques de trafic 3 (TRAPPC3), ST3 bêta-galactoside alpha-2,3- sialyltransférase 3 (ST3GAL3), motif YLP contenant 1 (YLPM1), protéine d'activation tyrosine 3-monooxygénase/tryptophane 5-monooxygénase zêta (YWHAZ) chez un patient comprenant l'étape de détection des auto-anticorps dirigés contre lesdites protéines marqueurs dans un échantillon prélevé sur le patient, dans lequel la détection desdits auto-anticorps de l'étape de détection indique un cancer du côlon ou le risque de cancer du côlon.

2. Procédé selon la revendication 1 comprenant le diagnostic du cancer ou de polypes précancéreux, de préférence dans lequel il est établi une distinction desdits polypes précancéreux entre des polypes à haut risque et des polypes à faible risque, dans lequel lesdits polypes à haut risque comprennent des polypes villeux adénomateux, des polypes tubulovilleux adénomateux ou une co-occurrence de polypes tubulaires adénomateux et de polypes tubulovilleux.

3. Procédé selon la revendication 1 ou 2 comprenant une distinction entre des conditions de bonne santé et un cancer.

4. Procédé selon l'une quelconque des revendications 1 à 3, dans lequel l'étape de détection d'auto-anticorps contre lesdites protéines marqueurs comprend la comparaison dudit signal de détection avec des signaux de détection d'un témoin en bonne santé et la comparaison desdits signaux de détection, dans lequel une augmentation du signal de détection indique un cancer du côlon ou ledit risque de cancer du côlon.

5. Procédé selon l'une quelconque des revendications 1 à 4, dans lequel l'étape de détection d'auto-anticorps contre lesdites protéines marqueurs comprend la comparaison dudit signal de détection avec des signaux de détection d'un ou plusieurs échantillons témoins connus de maladies sélectionnées parmi le cancer, les polypes précancéreux et/ou un témoin en bonne santé, dans lequel les signaux de contrôle sont utilisés pour obtenir un modèle de signal dépendant du marqueur comme classificateur d'indication et les signaux dépendant du marqueur du patient sont comparés et/ou ajustés sur ledit modèle, obtenant ainsi des informations sur la maladie diagnostiquée.

6. Procédé selon l'une quelconque des revendications 1 à 5, dans lequel l'étape de détection d'auto-anticorps contre lesdites protéines marqueurs comprend la comparaison dudit signal de détection avec des signaux de détection d'un témoin souffrant d'un cancer ou de polypes précancéreux et la comparaison desdits signaux de détection, dans lequel un signal de détection issu de l'échantillon d'un patient selon une amplitude d'au moins 60 %, de préférence d'au moins 80 %, du témoin souffrant d'un cancer indique un cancer du côlon ou ledit risque de cancer du côlon.

7. Procédé selon l'une quelconque des revendications 1 à 5, dans lequel l'étape de détection d'auto-anticorps contre lesdites protéines marqueurs comprend la comparaison dudit signal de détection avec des signaux de détection d'un témoin souffrant d'un cancer ou de polypes précancéreux et la comparaison desdits signaux de détection, dans lequel un signal de détection dans au moins 60 % des marqueurs utilisés indique un cancer du côlon ou ledit risque de cancer du côlon.

8. Procédé selon la revendication 7, dans lequel un signal de détection dans au moins 75 % des marqueurs utilisés indique un cancer du côlon ou ledit risque de cancer du côlon.

9. Utilisation d'un kit adapté pour détecter des auto-anticorps contre toute combinaison de marqueurs telle que définie dans la revendication 1, dans laquelle le kit comprend des agents diagnostiques comprenant des protéines marqueurs sélectionnées parmi ArsA Arsenite Transporter, ATP-Binding, Homolog 1 (ASNA1) et au moins 1 des protéines marqueurs sélectionnées parmi les marqueurs adaptateur et échafaudage du protéasome Ecm29 (KIAA0368, également appelé ECPAS), sous-unité catalytique 1 de queueine ARNt-ribosyltransférase (QTRT1), signaux de localisation nucléaire 1 du domaine SAM et du domaine SH3 (SAMSN1), glycoprotéine Ib sous-unité plaquettaire bêta (GP1BB), homologue à l'actine 6B (ACTL6B), af-tiphilin (AFTPH), protéine d'ancrage A-kinase 9 (AKAP9), facteur d'échange de nucléotides Rho guanine 1 (ARHGEF1), ligand de chimiokine motif CC 28 (CCL28), cadhérine 2 (CDH2), C- terminale de la kinase Src (CSK), dCTP pyrophosphatase 1 (DCTPP1), DEAD-box hélicase 3 Y-linked (DDX3Y), famille de protéines du choc thermique DnaJ (Hsp40) membre C10 (DNAJC10), EMAP like 2 (EML2), protéine de liaison ARF 1 associée à golgi, gamma adaptin ear (GGA1), sous-unité alpha du complexe multienzymatique trifonctionnel hydroxy-acyl-CoA déshydrogénase (HADHA), domaine de liaison nucléosomique du groupe à haute mobilité 2 (HMGN2), modificateur de type ubiquitine ISG15 (ISG15), répétition riche en leucine et motif alpha stérile contenant 1 (LRSAM1), protéine associée du composant 3 du complexe d'entretien des minichromosomes (MCM3AP), porteur mitochondrial 2 (MTCH2), sous-unité de caryophérine alpha 2 (KPNA2), polycystine 1 like 1, interagissant sur le canal potentiel du récepteur transitoire (PKD1L1), élément transposable pogo dérivé avec domaine ZNF (POGZ), protéine arginine méthyltransférase 1 (PRMT1), facteur de traitement pré-ARNm 4B (PRPF4B), récepteur kinase 6 couplé à la protéine G (GRK6), protéine de liaison à l'ARNm SERPINE1 1 (SERBP1), complexe de particules protéiques de trafic 3 (TRAPPC3), ST3 bêta-galactoside alpha-2,3- sialyltransférase 3 (ST3GAL3), motif YLP contenant 1 (YLPM1), protéine d'activation tyrosine 3-monooxygénase/tryptophane 5-monooxygénase zêta (YWHAZ) ou des fragments antigéniques de ceux-ci pour se lier aux auto-anticorps dans un échantillon ; dans un procédé selon l'une quelconque des revendications 1 à 8.

10. Utilisation selon la revendication 9, le kit comprenant en outre des données de signal pour des échantillons témoins avec des conditions connues sélectionnées parmi le cancer ou un polype précancéreux, et/ou des témoins en bonne santé.

11. Utilisation selon la revendication 9 ou 10, le kit comprenant en outre des données d'étalonnage ou d'apprentissage pour analyser lesdits marqueurs fournis dans le kit en vue de diagnostiquer le cancer du côlon ou de distinguer des maladies sélectionnées parmi les conditions de bonne santé, le cancer ou les polypes précancéreux.

12. Utilisation selon l'une quelconque des revendications 9 à 11, dans laquelle lesdits agents diagnostiques sont immobilisés sur un support solide.

13. Utilisation selon l'une quelconque des revendications 9 à 12, le kit comprenant au maximum 3 000 agents diagnostiques.

14. Utilisation selon l'une quelconque des revendications 9 à 12, le kit comprenant au maximum 2 000 agents diagnostiques.

15. Utilisation selon la revendication 14, le kit comprenant au maximum 1 000 agents diagnostiques.

16. Utilisation selon la revendication 15, le kit comprenant au maximum 200 agents diagnostiques.
